# EUROPÄISCHE PATENTSCHRIFT

(11) **EP 1 740 673 B1**
(45) Veröffentlichungstag und Bekanntmachung des Hinweises auf die Patenterteilung: **02.12.2009**
(21) Anmeldenummer: 05716040.0
(22) Anmeldetag: 14.03.2005
(51) Int. Cl.: C09K 11/06, H05B 33/14, H01L 51/30, C07D 487/04

(54) **TRIAZOLDERIVATE UND VERWENDUNG VON TRIAZOLDERIVATEN IN ORGANISCHEN LEUCHTDIODEN (OLEDS)**
TRIAZOLE DERIVATIVES AND USE THEROF IN ORGANIC LIGHT-EMITTING DIODES(OLEDS)
DERIVES DE TRIAZOL ET UTILISATION DE DERIVES DE TRIAZOLDANS DES DIODES ORGANIQUES ELECTROLUMINESCENTES

(30) Priorität: 23.03.2004 DE 102004014534
(43) Veröffentlichungstag der Anmeldung: 10.01.2007
(73) Patentinhaber: BASF SE, 67056 Ludwigshafen (DE)
(72) Erfinder: GESSNER, Thomas, 69120 Heidelberg (DE); LENNARTZ, Christian, 67105 Schifferstadt (DE); SCHMIDT, Hans-Werner, 95444 Bayreuth (DE); THELAKKAT, Mukundan, 95445 Bayreuth (DE); BAETE, Markus, 95508 Kulmain (DE)
(74) Vertreter: Isenbruck, Günter
(86) Internationale Anmeldenummer: PCT/EP2005/002697
(87) Internationale Veröffentlichungsnummer: WO 2005/093007

(56) Entgegenhaltungen:
- EP-A- 1 026 222
- DE-A1- 2 749 902
- DE-A1- 3 304 330
- US-A- 3 817 990
- US-A- 4 157 443
- US-A- 4 739 053
- NARITA S ET AL: "APPLICATION OF FLUORESCENT TRIAZOLES TO ANALYTICAL CHEMISTRY. I. DETERMINATION OF AROMATIC PRIMARY AMINE WITH 2,4,6-TRIAMINOPYRIMIDINE AS A REAGENT" CHEMICAL AND PHARMACEUTICAL BULLETIN, PHARMACEUTICAL SOCIETY OF JAPAN, TOKYO, JP, Bd. 33, Nr. 11, 1985, Seiten 4928-4934, XP008049633 ISSN: 0009-2363

## Beschreibung

Die vorliegende Erfindung betrifft die Verwendung von Triazolderivaten ausgewählt aus der Gruppe bestehend aus Triazolopyrimidinderivaten und Triazolouracilderivaten in organischen Leuchtdioden (OLEDs), ein OLED enthaltend mindestens eines der genannten organischen Triazolderivate, eine Licht-emittierende Schicht enthaltend mindestens eines der genannten Triazolderivate, ein OLED enthaltend die erfindungsgemäße Licht-emittierende Schicht, eine Vorrichtung, die ein erfindungsgemäßes OLED enthält sowie spezielle neue Triazolderivate.

In organischen Leuchtdioden (OLED) wird die Eigenschaft von Materialien ausgenutzt, Licht zu emittieren, wenn sie durch elektrischen Strom angeregt werden. OLEDs sind insbesondere interessant als Alternative zu Kathodenstrahlröhren und Flüssigkristalldisplays zur Herstellung von Flachbildschirmen. Aufgrund der sehr kompakten Bauweise und des intrinsisch niedrigeren Stromverbrauchs eignen sich Vorrichtungen enthaltend OLEDs insbesondere für mobile Anwendungen, zum Beispiel für Anwendungen in Handys, Laptops usw.

Es wurden zahlreiche Materialien zur Anwendung in OLEDs vorgeschlagen. Der Einsatz von speziellen Triazolderivaten in OLEDs wurde ebenfalls bereits erwähnt.

EP-A 0 710 655 betrifft grün emittierende Benzotriazol-Metallkomplexe zur Verwendung in Licht-emittierenden Elementen. Die eingesetzten Benzotriazol-Metallkomplexe weisen die folgende Formel auf:

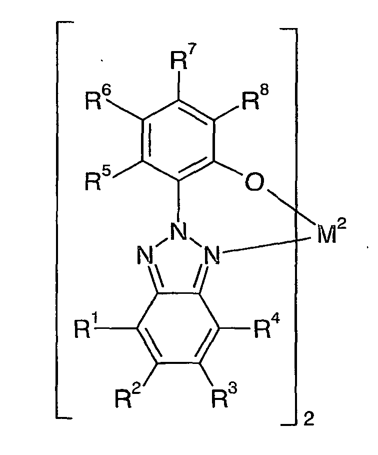

EP-A 0 875 947 betrifft blau emittierende organische LEDs mit Oxadiazol-, Thiadiazol- oder Triazolderivaten in der elektrolumineszierenden Schicht. Diese Derivate weisen die folgende Formel auf:

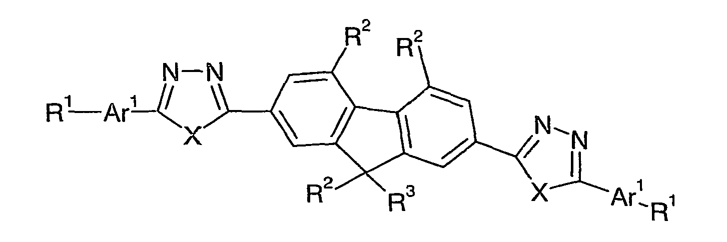

EP-A 0 704 436 betrifft Triazolderivate und daraus hergestellte organische elektrolumineszierende Elemente. Die Triazolderivate weisen die folgende Formel auf:

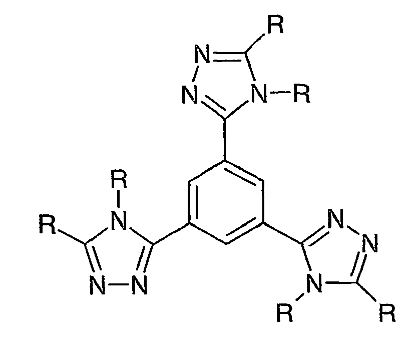

Die Triazolderivate werden dabei bevorzugt in der Elektronentransportschicht eingesetzt. Bei Variation der Reste sind die Triazolderivate grundsätzlich jedoch auch für Licht-emittierende Materialien geeignet.
Bei den vorstehend genannten Triazolderivaten handelt es sich um spezielle Verbindungen, wobei der Einsatz von Triazolopyrimidinderivaten und Triazolouracilderivaten in OLEDs nicht erwähnt wird.

Triazolopyrimidinderivate und Triazolouracilderivate sind im Stand der Technik bereits bekannt.

So betrifft DE-A 30 01424 v-Triazolyl-[4,5-d]-pyrimidine der allgemeinen Formel

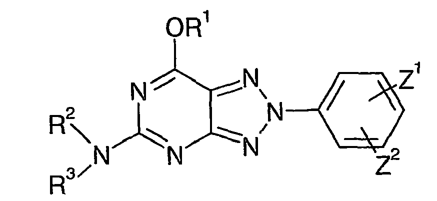

DE-A 27 49 902 betrifft ebenfalls v-Triazolyl-[4,5-d]-pyrimidine. Diese Verbindungen weisen die allgemeine Formel 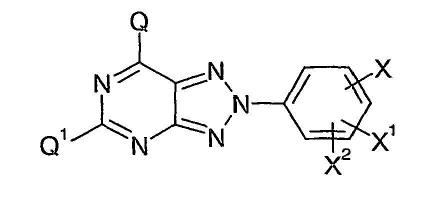 auf.

DE-A 25 54 027 betrifft Triazolylverbindungen der allgemeinen Formel 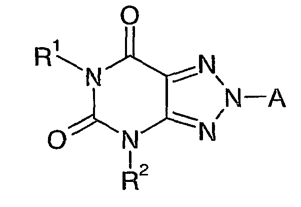 worin A einen substituierten Phenylrest darstellt.

Die in DE-A 30 01424, DE-A 27 49 902 und DE-A 25 54 027 offenbarten Verbindungen zeigen in gelöstem oder fein verteiltem Zustand eine mehr oder weniger ausgeprägte Fluoreszenz und können zum optischen Aufhellen von organischen Materialien eingesetzt werden.

Keines der drei vorstehend genannten Dokumente betrifft eine Elektrolumineszenz der offenbarten Triazolderivate bzw. deren Einsatz in OLEDs.

WO 03/105538 betrifft 2H-Benzotriazole und deren Einsatz in organischen Leuchtdioden. Die Benzotriazole weisen mindestens eine 2H-Benzotriazoleinheit der folgenden Formel auf: 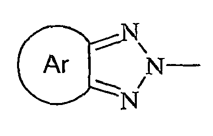 worin Ar ein Aryl- oder Heteroarylrest sein kann. Zwar wird die Grundstruktur von Triazolopyrimidinderivaten in WO 03/105538 unter anderem erwähnt, jedoch ohne Angabe konkreter Substituenten.

Obwohl bereits Verbindungen bekannt sind, die im blauen, roten und grünen Bereich des elektromagnetischen Spektrums Elektrolumineszenz zeigen, ist die Bereitstellung von weiteren Verbindungen wünschenswert. Unter Elektrolumineszenz ist sowohl Elektrofluoreszenz als auch Elektrophosphoreszenz zu verstehen.

Aufgabe der vorliegenden Anmeldung ist daher die Bereitstellung von Verbindungen, die zu Elektrolumineszenz im sichtbaren Bereich des elektromagnetischen Spektrums, insbesondere im blauen bis blau-grünen Bereich, geeignet sind.

Diese Aufgabe wird durch die Verwendung von Triazolderivaten ausgewählt aus der Gruppe bestehend aus Triazolopyrimidinderivaten, enthaltend mindestens einen Substituenten ausgewählt aus der Gruppe bestehend aus OH, O-Alkyl, O-Aryl, Halogen und Amino, und Triazolouracilderivaten in organischen Leuchtdioden (OLEDs) gelöst.

Es wurde gefunden, dass die Triazolopyrimidinderivate, die mindestens einen Substituenten ausgewählt aus der Gruppe bestehend aus OH, O-Aryl, Halogen und Amino aufweisen, und Triazolouracilderivate Elektrolumineszenz im sichtbaren Bereich des elektromagnetischen Spektrums zeigen und somit als Emittermoleküle in OLEDs eingesetzt werden können.

Ein weiterer Gegenstand der vorliegenden Anmeldung ist daher die Verwendung der Triazolopyrimidinderivate und Triazolouracilderivate gemäß der vorliegenden Anmeldung als Emittermoleküle in OLEDs.

Es ist ebenfalls möglich, die Triazolopyrimidinderivate und Triazolouracilderivate gemäß der vorliegenden Anmeldung als Wirtsmoleküle (Matrix) in der Emitterschicht einzusetzen.

Ein weiterer Gegenstand der vorliegenden Anmeldung ist daher die Verwendung der Triazolopyrimidinderivate und Triazolouracilderivate gemäß der vorliegenden Anmeldung als Wirtsmoleküle (Matrix) in der Emitterschicht.

Des Weiteren ist es denkbar, die genannten Triazolderivate auch in anderen Schichten eines OLEDs, zum Beispiel in der Elektronentransportschicht, einzusetzen.

Bevorzugt sind die erfindungsgemäß verwendeten Triazolderivate ausgewählt aus der Gruppe bestehend aus Triazolopyrimidinderivaten und Triazolouracilderivaten ausgewählt aus Verbindungen der Strukturformeln I, II, III und IV: 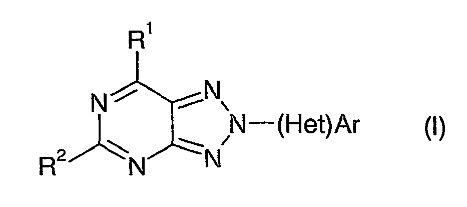 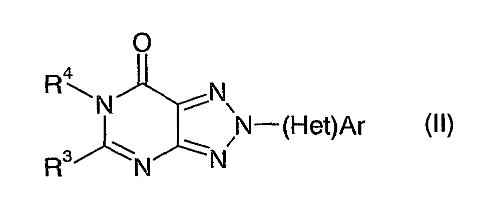 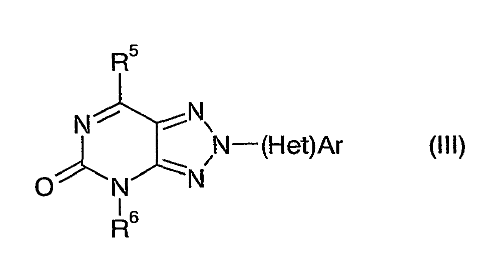 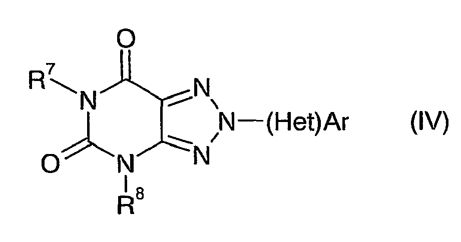 worin die Symbole die folgenden Bedeutungen aufweisen:
- R¹, R²: unabhängig voneinander H, Alkyl, Aryl, Heteroaryl, OH, O-Alkyl, O-Aryl, Halogen oder Amino, wobei mindestens einer der Substituenten R¹ oder R² OH, O-Alkyl, O-Aryl, Halogen oder Amino, bevorzugt Amino, bedeutet;
- R³, R⁵: unabhängig voneinander H, Alkyl, Aryl, Heteroaryl, OH, O-Alkyl, O-Aryl, Halogen oder Amino;
- R⁴, R⁶, R⁷, R⁸: unabhängig voneinander H, Alkyl, Aryl oder Heteroaryl;
oder
- R³ und R⁴: bilden gemeinsam mit den Atomen, an die sie gebunden sind, einen 4- bis 8-gliedrigen Ring, der gegebenenfalls weitere Heteroatome enthält und gesättigt oder ungesättigt und unsubstituiert oder substituiert ist;
- (Het)Ar: Aryl oder Heteroaryl.

Im Sinne der vorliegenden Anmeldung haben die im Folgenden aufgeführten Begriffe die folgenden Bedeutungen:

Unter Aryl ist ein Rest mit einem Grundgerüst von 6 bis 30 Kohlenstoffatomen, bevorzugt 6 bis 18 Kohlenstoffatomen zu verstehen, der aus einem aromatischen Ring oder mehreren kondensierten aromatischen Ringen aufgebaut ist. Geeignete Grundgerüste sind zum Beispiel Phenyl, Naphthyl, Anthracenyl oder Phenanthrenyl. Dieses Grundgerüst kann unsubstituiert sein (das heißt, dass alle Kohlenstoffatome, die substituierbar sind, Wasserstoffatome tragen), oder an einer, mehreren oder allen substituierbaren Positionen des Grundgerüstes substituiert sein. Geeignete Substituenten sind zum Beispiel Alkylreste, bevorzugt Alkylreste mit 1 bis 8 Kohlenstoffatomen, besonders bevorzugt Methyl, Ethyl, i-Propyl oder t-Butyl, Arylreste, bevorzugt C₆-Arylreste, die wiederum substituiert oder unsubstituiert sein können, Styrylreste, Heteroarylreste, bevorzugt Heteroarylreste, die mindestens ein Stickstoffatom enthalten, besonders bevorzugt Pyridylreste, Alkenylreste, bevorzugt Alkenylreste, die eine Doppelbindung tragen, besonders bevorzugt Alkenylreste mit einer Doppelbindung und 1 bis 8 Kohlenstoffatomen, oder Gruppen mit Donor- oder Akzeptorwirkung. Unter Gruppen mit Donorwirkung sind Gruppen zu verstehen, die einen +I-und/oder +M-Effekt aufweisen, und unter Gruppen mit Akzeptorwirkung sind Gruppen zu verstehen, die einen -I- und/oder -M-Effekt aufweisen. Geeignete Gruppen mit Donor- oder Akzeptorwirkung sind Halogenreste, bevorzugt F, Cl, Br, besonders bevorzugt F, Alkoxyreste, Carbonylreste, Esterreste, Aminreste, Amidreste, CH₂F-Gruppen, CHF₂-Gruppen, CF₃-Gruppen, CN-Gruppen, Thiogruppen oder SCN-Gruppen. Ganz besonders bevorzugt tragen die Arylreste Substituenten ausgewählt aus der Gruppe bestehend aus Methyl, F, Cl und Alkoxy, oder die Arylreste sind unsubstituiert. Bevorzugt ist der Arylrest ein C₆-Arylrest oder ein Naphthylrest, der gegebenenfalls mit mindestens einem der vorstehend genannten Substituenten substituiert ist. Besonders bevorzugt weist der C₆-Arylrest keinen, einen oder zwei der vorstehend genannten Substituenten auf, wobei der eine Substituent bevorrzugt in para-Position zur weiteren Verknüpfungsstelle des Arylrests angeordnet ist und - im Falle von zwei Substituenten - diese jeweils in ortho-Position zur weiteren Verknüpfungsstelle des Arylrestes angeordnet sind. Ganz besonders bevorzugt ist der C₆-Arylrest ein unsubstituierter Phenylrest. Der Naphthylrest ist bevorzugt 1-Naphthyl oder 2-Naphthyl.

Unter Heteroaryl sind Heteroarylreste zu verstehen, die sich von den vorstehend genannten Arylresten dadurch unterscheiden, dass in dem Grundgerüst der Arylreste mindestens ein Kohlenstoffatom durch ein Heteroatom ersetzt ist. Bevorzugte Heteroatome sind N, O und S. Ganz besonders bevorzugt sind ein oder zwei Kohlenstoffatome des Grundgerüstes der Arylreste durch Heteroatome ersetzt. Insbesondere bevorzugt ist das Grundgerüst ausgewählt aus Systemen wie Pyridyl, Imidazolyl, cyclischen Estern, cyclischen Amiden und fünfgliedrigen Heteroaromaten wie Thienyl, Pyrrolyl und Furyl. Das Grundgerüst kann an einer, mehreren oder allen substituierbaren Positionen des Grundgerüsts substituiert sein. Geeignete Substituenten sind dieselben, die bereits bezüglich Aryl genannt wurden. Besonders bevorzugt ist Thienyl.

Unter Alkyl ist ein Alkylrest mit 1 bis 20 Kohlenstoffatomen, bevorzugt 1 bis 10 Kohlenstoffatomen, besonders bevorzugt 1 bis 8 Kohlenstoffatomen, ganz besonders bevorzugt 1 bis 4 Kohlenstoffatomen zu verstehen. Dieser Alkylrest kann verzweigt oder unverzweigt sein und gegebenenfalls mit einem oder mehreren Heteroatomen, bevorzugt N, O oder S unterbrochen sein. Weiterhin kann der Alkylrest oder Alkylgruppe ein C₃- bis C₈-Cycloalkylrest, bevorzugt ein C₅- oder C₆-Cycloalkylrest sein, der gegebenenfalls mit einem oder mehreren Heteroatomen, bevorzugt N, O oder S unterbrochen sein kann, zum Beispiel Cyclopentyl und Cyclohexyl. Des Weiteren kann dieser Alkylrest mit einem oder mehreren bezüglich der Arylgruppen genannten Substituenten, insbesondere Halogenresten, bevorzugt F, Cl, Br, besonders bevorzugt F, substituiert sein. Es ist ebenfalls möglich, dass der Alkylrest eine oder mehrere Arylgruppen trägt. Dabei sind alle der vorstehend aufgeführten Arylgruppen geeignet. Besonders bevorzugt sind die Alkylreste ausgewählt aus der Gruppe bestehend aus Methyl, Ethyl, i-Propyl, n-Propyl, i-Butyl, n-Butyl, t-Butyl, sec-Butyl, i-Pentyl, n-Pentyl, sec-Pentyl, neo-Pentyl, n-Hexyl, i-Hexyl, sec-Hexyl, Cyclopentyl und Cyclohexyl. Ganz besonders bevorzugt sind Methyl, Ethyl, i-Propyl, t-Butyl und n-Hexyl, insbesondere bevorzugt ist Methyl.

Unter O-Alkyl ist ein Alkoxyrest zu verstehen, der die allgemeine Formel -OR⁹ aufweist. R⁹ stellt dabei einen Alkylrest dar, wie er vorstehend definiert wurde. Besonders bevorzugt ist O-Alkyl somit O-Methyl, O-Ethyl, O-ⁱPropyl, O-^{t}Butyl und O-ⁿHexyl. Ganz besonders bevorzugt ist O-Methyl.

Unter O-Aryl ist ein Aryloxyrest zu verstehen, der die allgemeine Formel -OR¹⁰ aufweist. R¹⁰ stellt dabei einen Arylrest dar, wie er vorstehend definiert ist. Ganz besonders bevorzugt ist O-Aryl, O-Phenyl, wobei der Phenylrest unsubstituiert ist.

Unter Halogen ist bevorzugt ein Substituent ausgewählt aus der Gruppe F, Cl und Br zu verstehen. Besonders bevorzugt ist Halogen Cl oder Br, ganz besonders bevorzugt Cl.

Unter Amino ist eine Aminogruppe der Formel NR¹¹R¹² zu verstehen. R¹¹ und R¹² sind dabei unabhängig voneinander H, Alkyl, Aryl oder Heteroaryl. Bevorzugte Alkyl-, Aryl- und Heteroarylgruppen sind bereits vorstehend erwähnt. Ganz besonders bevorzugt ist Dimethylamino. Des Weiteren können R¹¹ und R¹² gemeinsam mit dem N-Atom der Aminogruppe einen cyclischen Rest bilden. Dabei handelt es sich bevorzugt um einen fünf- oder sechsgliedrigen cyclischen Rest. Dieser kann neben dem Stickstoffatom einen oder mehrere weitere Heteroatome ausgewählt aus N, O und S enthalten. Ganz besonders bevorzugte cyclische Aminoreste sind Morpholino, Pyrrolidino und Piperidino. Ganz besonders bevorzugte Aminogruppen sind Dimethylamino, Morpholino, Pyrrolidino und Piperidino.

Unter (Het)Ar ist ein Arylrest oder ein Heteroarylrest zu verstehen. Geeignete Arylreste und Heteroarylreste sind bereits vorstehend genannt. Bevorzugt ist (Het)Ar ein Rest der Formel 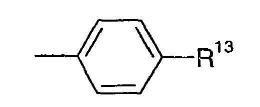 worin R¹³ H, Alkyl, O-Alkyl, S-Alkyl, Aryl, O-Aryl, S-Aryl oder Alkenylaryl bedeutet. Dabei haben die Begriffe Alkyl, O-Alkyl, Aryl und O-Aryl die vorstehend angegebenen Bedeutungen. Bevorzugt ist der Rest R¹³ H, OCH₃, Phenyl, das substituiert oder unsubstituiert sein kann, besonders bevorzugt unsubstituiertes Phenyl, oder Styryl, das unsubstituiert ist.

Unter S-Alkyl ist ein Rest der allgemeinen Formel S-R¹⁴ zu verstehen. R¹⁴ ist dabei ein Alkylrest, wie er vorstehend definiert wurde. Bevorzugt ist S-Alkyl somit S-Methyl, S-Ethyl, S-ⁱPropyl, S-^{t}Butyl und S-ⁿHexyl, besonders bevorzugt ist S-Methyl.

Unter S-Aryl ist ein Rest der allgemeinen Formel S-R¹⁵ zu verstehen. R¹⁵ ist dabei ein Arylrest, wie er vorstehend definiert wurde. Bevorzugt ist S-Aryl somit S-Phenyl, wobei der Phenylrest unsubstituiert ist.

In einer bevorzugten Ausführungsform betrifft die vorliegende Erfindung Triazolderivate der Formel I, worin
- R¹: Cl, OH, OCH₃, OPh oder Morpholino bedeutet, bevorzugt OH oder OCH₃, Cl oder Morpholino, besonders bevorzugt Cl oder Morpholino;
und/oder
- R²: OH, OCH₃, OPh, Piperidino, Pyrrolidino, Morpholino oder N(CH₃)₂ bedeutet, bevorzugt OH, N(CH₃)₂, Piperidino, Pyrrolidino oder Morpholino, besonders bevorzugt N(CH₃)₂.

In einer weiteren bevorzugten Ausführungsform betrifft die vorliegende Erfindung Triazolderivate der Formel II oder III, worin
- R³, R⁵: unabhängig voneinander OH, OCH₃, OPh, Piperidino, Pyrrolidino, Morpholino oder N(CH₃)₂ bedeuten, bevorzugt OH, N(CH₃)₂, Piperidino, Pyrrolidino oder Morpholino;
und/oder
- R⁴, R⁶: unabhängig voneinander H, CH₃ oder Phenyl bedeuten, bevorzugt H.

In einer weiteren bevorzugten Ausführungsform betrifft die vorliegende Erfindung Triazolderivate der Formel IV, worin
- R⁷ und R⁸: unabhängig voneinander H, CH₃ oder Phenyl, bevorzugt CH₃, bedeuten.

In einer weiteren bevorzugten Ausführungsform betrifft die vorliegende Erfindung Triazolderivate der Formel II, worin
- R³ und R⁴: gemeinsam mit den Atomen, an die sie gebunden sind, einen 5- bis 7-gliedrigen Ring bilden, der gegebenenfalls weitere Heteroatome, bevorzugt ausgewählt aus S, N und O, besonders bevorzugt ein weiteres N-Atom, enthält und gesättigt oder ungesättigt, bevorzugt gesättigt, ist, und unsubstituiert oder substituiert ist. Besonders bevorzugt weist der Ring einen Substituenten ausgewählt aus Alkyl und Aryl an dem einen weiteren N-Atom auf, während die übrigen Atome des Rings unsubstituiert sind (d.h. Wasserstoffatome tragen).

Ganz besonders bevorzugt weisen die Verbindungen der Formel II, worin R³ und R⁴ gemeinsam mit den Atomen, an die sie gebunden sind, einen 5- bis 7-gliedrigen Ring bilden, die folgenden Strukturformeln auf: 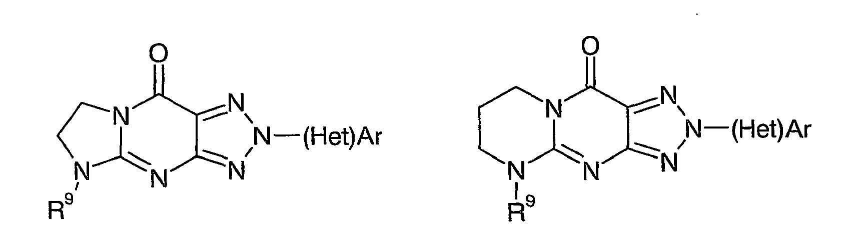 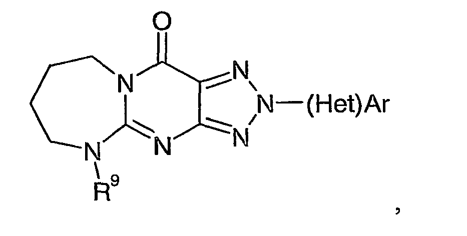 worin R⁹ Alkyl oder Aryl bedeutet und (Het)Ar bereits vorstehend definiert ist. Bevorzugt bedeutet (Het)Ar

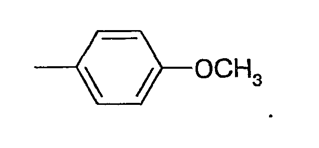

Die vorstehend genannten Triazolderivate sind hervorragend für den Einsatz in organischen Leuchtdioden (OLEDs) geeignet. Ein weiterer Gegenstand der vorliegenden Erfindung sind daher organische Leuchtdioden enthaltend mindestens ein Triazolderivat, wie es gemäß der vorliegenden Anmeldung definiert ist. Bevorzugt werden die Triazolderivate gemäß der vorliegenden Anmeldung in der Elektronen-leitenden Schicht oder der Licht-emittierenden Schicht als Emittermoleküle in OLEDs eingesetzt. Besonders bevorzugt ist der Einsatz der Triazolderivate gemäß der vorliegenden Anmeldung in der Licht-emittierenden Schicht von OLEDs als Emittermoleküle.

Die vorstehend genannten erfindungsgemäß verwendeten Triazolderivate können nach dem Fachmann bekannten Verfahren hergestellt werden.

Übliche Verfahren sind zum Beispiel die Kupplung von geeigneten Aminen mit geeigneten Diazoniumsalzen mit anschließendem oxidativen Ringschluss.

Triazolderivate der Formel I können somit nach dem folgenden Verfahren hergestellt werden:

### a) Kupplung eines Amins der Formel V

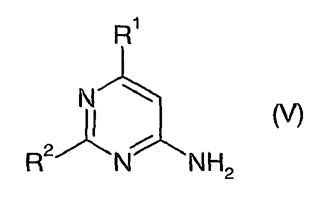

worin R¹ und R² die vorstehend angegebenen Bedeutungen aufweisen, mit einem Diazoniumsalz der Formel VI 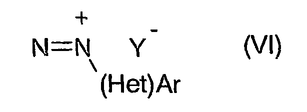 worin (Het)Ar die vorstehend angegebene Bedeutung aufweist und Y⁻ ein Anion bedeutet, zu einer Azoverbindung der Formel VII 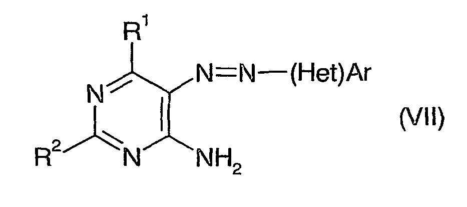 worin R¹, R² und (Het)Ar die vorstehend angegebenen Bedeutungen aufweisen;

### b) oxidativer Ringschluss der Azoverbindung der Formel VII zu den gewünschten Triazolderivaten der Formel I.

Die Verbindungen der Formel II können nach dem folgenden Verfahren erhalten werden:

### a) Kupplung eines Amins der Formel VIII

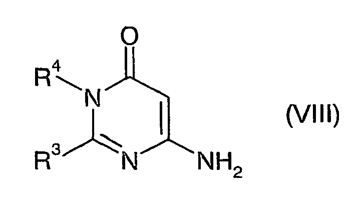

worin R³ und R⁴ die vorstehend angegebenen Bedeutungen aufweisen, mit einem Diazoniumsalz der Formel VI 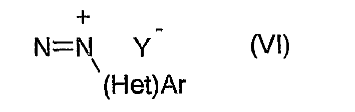 worin (Het)Ar die vorstehend genannte Bedeutung aufweist und Y⁻ ein Anion bedeutet, wobei eine Azoverbindung der Formel IX erhalten wird 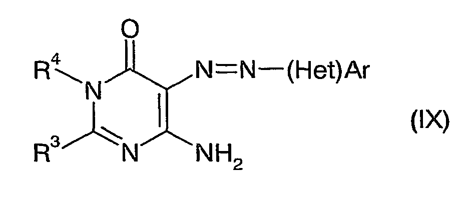 worin R³, R⁴ und (Het)Ar die vorstehend angegebenen Bedeutungen aufweisen;

### b) oxidativer Ringschluss der Azoverbindung der Formel IX, wobei ein Triazolderivat der Formel II erhalten wird.

Die Verbindungen der Formel III können nach dem folgenden Verfahren erhalten werden:

### a) Kupplung eines Amins der Formel X

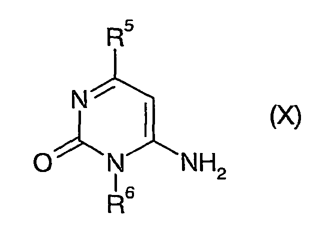

worin R⁵ und R⁶ die vorstehend angegebenen Bedeutungen aufweisen, mit einem Diazoniumsalz der Formel VI 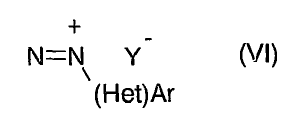 worin (Het)Ar die vorstehend genannte Bedeutung aufweist und Y⁻ ein Anion bedeutet, wobei eine Azoverbindung der Formel XI erhalten wird 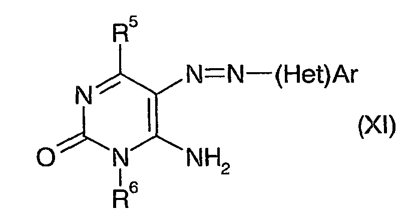 worin R⁵ und R⁶ und (Het)Ar die vorstehend angegebenen Bedeutungen aufweisen;

### b) oxidativer Ringschluss der Azoverbindung der Formel XI, wobei ein Triazolderivat der Formel III erhalten wird.

Die Verbindungen der Formel IV können nach dem folgenden Verfahren erhalten werden:

### a) Kupplung eines Amins der Formel XII

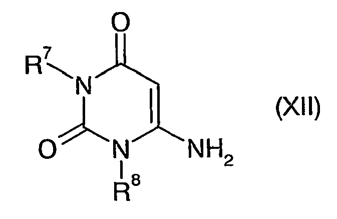

worin R⁵ und R⁶ die vorstehend angegeben Bedeutungen aufweisen, mit einem Diazoniumsalz der Formel VI 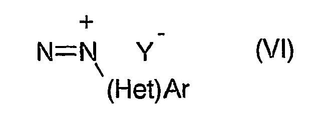 worin (Het)Ar die vorstehend angegebene Bedeutung aufweist und Y⁻ ein Anion bedeutet, wobei eine Azoverbindung der Formel XIII 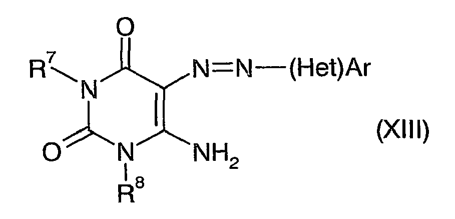 entsteht, worin R⁷, R⁸ und (Het)Ar die vorstehend angegebenen Bedeutungen aufweisen;

### b) oxidativer Ringschluss der Azoverbindung der Formel XIII, wobei ein Triazolderivat der Formel IV erhalten wird.

### Schritt a)

Die als Kupplungskomponenten verwendeten Amine der Formeln V, VIII, X und XII sind aus der Literatur bekannt oder können in Analogie zu Literatur-bekannten Verfahren hergestellt werden. Siehe dazu J. Am. Chem. Soc. 73 (1951), 2864, J. Chem. Soc. 1962, 3172, Chem. Pharm. Bull. Japan 13 (1965), 557.
Die Diazoniumsalze der Formel VI können in üblicher Weise durch Diazotierung der entsprechenden Aniline erhalten werden. Diese Aniline sind bekannt oder können in Analogie zu dem Fachmann bekannten Verfahren hergestellt werden, zum Beispiel aus Chlornitrobenzolen durch Umsetzung mit einem geeigneten Alkohol und anschließende Reduktion der Nitrogruppe zur Aminogruppe. Das Anion in der Diazoniumsalze der Formel VI ist bevorzugt ein Halogenanion, zum Beispiel Chlorid oder Bromid, ein Sulfation oder ein Tetrafluoroboration.

Die Kupplung eines Amins der Formel V, VIII, X oder XII mit einem Diazoniumsalz der Formel VI erfolgt nach dem Fachmann bekannten Verfahren. Bevorzugt wird die Kupplung einer Temperatur von -10 bis +20°C, besonders bevorzugt von 0 bis 10°C vorgenommen.

### Schritt b)

Der oxidative Ringschluss wird im Allgemeinen nach dem Fachmann bekannten Verfahren durchgeführt, zum Beispiel nach einem Verfahren, wie in US 2,543,333 beschrieben. Der oxidative Ringschluss kann durch Einwirkung von verschiedenen Oxidationsmitteln bewirkt werden. Geeignete Oxidationsmittel sind dem Fachmann bekannt. Beispielsweise sind Chromsäure, Alkalibichromate, Wasserstoffperoxid, Bleitetraacetat, Kaliumferricyanid, Ferrichlorid und Kupfer(II)sulfat geeignet.

Der oxidative Ringschluss kann in sauren oder basischen Lösungsmitteln durchgeführt werden. In sauren Lösungsmitteln, zum Beispiel wässriger Essigsäure, werden vorzugsweise Alkalibichromate, Wasserstoffperoxid oder Bleitetraacetat als Oxidationsmittel eingesetzt. In basischen Lösungsmitteln, zum Beispiel Pyridin-Wassergemischen, wird vorzugsweise Kaliumferricyanid oder Kupfer(II)sulfat verwendet. Besonders bevorzugt wird der oxidative Ringschluss mit Kupfer(II)sulfat in einem Pyridin-Wassergemisch bewirkt. Die Oxidation mit Kuper(II)salzen, wie Kupfer(II)sulfat oder Kupfer(II)chlorid, lässt sich des Weiteren in Methanol oder Methanol-Wassergemischen in Gegenwart von Ammonium- oder Aminsalzen, wie Mono- oder Dialkanolaminen durchführen.

Bevorzugt wird der oxidative Ringschluss bei einer Temperatur von 70 bis 100°C, besonders bevorzugt 90 bis 100°C durchgeführt.

Die erfindungsgemäß verwendeten Triazolderivate eignen sich zum Einsatz in OLEDs. Bevorzugt werden sie in der Elektronentransportschicht oder in der Licht-emittierenden Schicht eines OLEDs eingesetzt. Besonders bevorzugt werden die erfindungsgemäß verwendeten Triazolderivate als Emittersubstanzen in OLEDs eingesetzt, da sie Lumineszenz (Elektrolumineszenz) in sichtbaren Bereich des elektromagnetischen Spektrums zeigen. Mit Hilfe der erfindungsgemäß verwendeten Triazolderivate ist es möglich, Verbindungen bereit zu stellen, die Elektrolumineszenz im roten, grünen sowie im blauen Bereich des elektromagnetischen Spektrums, in Abhängigkeit von ihrem Substitutionsmuster, zeigen. Insbesondere ist es möglich, Verbindungen bereit zu stellen, die Elektrolumineszenz im blauen sowie im blau-grünen Bereich des elektromagnetischen Spektrums zeigen.

Ein weiterer Gegenstand der vorliegenden Anmeldung ist daher eine organische Leuchtdiode (OLED) enthaltend mindestens ein Triazolderivat ausgewählt aus der Gruppe bestehend aus Triazolopyrimidinderivaten und Triazolouracilderivaten gemäß der vorliegenden Anmeldung. Bevorzugte Triazolderivate ausgewählt aus der Gruppe bestehend aus Triazolopyrimidinderivaten und Triazolouracilderivaten sind bereits vorstehend genannt.

Organische Leuchtdioden (OLEDs) sind grundsätzlich aus mehreren Schichten aufgebaut:
1. Anode
2. Löcher-transportierende Schicht
3. Licht-emittierende Schicht
4. Elektronen-transportierende Schicht
5. Kathode

Die Triazolderivate ausgewählt aus der Gruppe bestehend aus Triazolopyrimidinderivaten und Triazolouracilderivaten werden bevorzugt in der Licht-emittierenden Schicht als Emittermoleküle eingesetzt. Ein weiterer Gegenstand der vorliegenden Anmeldung ist daher eine Licht-emittierende Schicht enthaltend mindestens ein Triazolderivat ausgewählt aus der Gruppe bestehend aus Triazolopyrimidinderivaten und Triazolouracilderivaten. Bevorzugte Triazolderivate ausgewählt aus der Gruppe bestehend aus Triazolopyrimidinderivaten und Triazolouracilderivaten sind bereits vorstehend genannt.
Die erfindungsgemäß verwendeten Triazolderivate ausgewählt aus der Gruppe bestehend aus Triazolopyrimidinderivaten und Triazolouracilderivaten können in Substanz - ohne weitere Zusätze - in der Licht-emittierenden Schicht vorliegen. Es ist jedoch ebenfalls möglich, dass neben den erfindungsgemäß eingesetzten Triazolderivaten ausgewählt aus der Gruppe bestehend aus Triazolopyrimidinderivaten und Triazolouracilderivaten weitere Verbindungen in der Licht-emittierenden Schicht vorliegen. Beispielsweise kann ein fluoreszierender Farbstoff anwesend sein, um die Emissionsfarbe der als Emittermoleküls eingesetzten organischen Verbindung zu verändern. Des Weiteren kann ein Verdünnungsmaterial eingesetzt werden. Dieses Verdünnungsmaterial kann ein Polymer sein, zum Beispiel Poly(N-vinylcarbazol) oder Polysilan. Das Verdünnungsmaterial kann jedoch ebenfalls ein kleines Molekül sein, zum Beispiel 4,4'-N,N'-Dicarbazolbiphenyl (CBP) oder tertiäre aromatische Amine. Wenn ein Verdünnungsmaterial eingesetzt wird, beträgt der Anteil der erfindungsgemäß eingesetzten Triazolderivate in der Licht-emittierenden Schicht im Allgemeinen weniger als 20 Gew.-%, bevorzugt 3 bis 10 Gew.-%. In einer Ausführungsform der vorliegenden Erfindung werden die erfindungsgemäß eingesetzten Triazolderivate ausgewählt aus der Gruppe bestehend aus Triazolopyrimidinderivaten und Triazolouracilderivaten in Substanz eingesetzt, wodurch eine aufwendige Coverdampfung der Triazolderivate mit einem Matrixmaterial (Verdünnungsmaterial oder fluoreszierender Farbstoff) vermieden wird. Dafür ist es wesentlich, dass die Triazolderivate ausgewählt aus der Gruppe bestehend aus Triazolopyrimidinderivaten und Triazolouracilderivaten im Festkörper lumineszieren. Die erfindungsgemäß eingesetzten organischen Verbindungen ausgewählt aus der Gruppe bestehend aus Triazolopyrimidinderivaten und Triazolouracilderivaten zeigen im Festkörper Lumineszenz. Somit enthält die Licht-emittierende Schicht in einer Ausführungsform mindestens ein Triazolderivat ausgewählt aus der Gruppe bestehend aus Triazolopyrimidinderivaten und Triazolouracilderivaten und kein Matrixmaterial ausgewählt aus Verdünnungsmaterial und fluoreszierendem Farbstoff.

Die einzelnen der vorstehend genannten Schichten des OLEDs können wiederum aus 2 oder mehreren Schichten aufgebaut sein. Beispielsweise kann die Löcher-transportierende Schicht aus einer Schicht aufgebaut sein in die aus der Elektrode Löcher injiziert werden und einer Schicht, die die Löcher von der Loch injizierenden Schicht weg in die Licht-emittierende Schicht transportiert. Die Elektronen-transportierende Schicht kann ebenfalls aus mehreren Schichten bestehen, zum Beispiel einer Schicht, worin Elektronen durch die Elektrode injiziert werden, und einer Schicht, die aus der Elektronen-injizierenden Schicht Elektronen erhält und in die Licht-emittierende Schicht transportiert. Diese genannten Schichten werden jeweils nach Faktoren wie Energieniveau, Temperaturresistenz und Ladungsträgerbeweglichkeit, sowie Energiedifferenz der genannten Schichten mit den organischen Schichten oder den Metallelektroden ausgewählt. Der Fachmann ist in der Lage, den Aufbau der OLEDs so zu wählen, dass er optimal an die erfindungsgemäß als Emittersubstanzen verwendeten organischen Verbindungen angepasst ist.

Um besonders effiziente OLEDs zu erhalten, sollte das HOMO (höchstes besetztes Molekülorbital) der Loch-transportierenden Schicht mit der Arbeitsfunktion der Anode angeglichen sein und das LUMO (niedrigstes unbesetztes Molekülorbital) der elektronentransportierenden Schicht sollte mit der Arbeitsfunktion der Kathode angeglichen sein.

Ein weiterer Gegenstand der vorliegenden Anmeldung ist ein OLED enthaltend mindestens eine erfindungsgemäße Licht-emittierende Schicht. Die weiteren Schichten in dem OLED können aus einem beliebigen Material aufgebaut sein, das üblicherweise in solchen Schichten eingesetzt wird und dem Fachmann bekannt ist.

Die Anode (1) ist eine Elektrode, die positive Ladungsträger bereitstellt. Sie kann zum Beispiel aus Materialien aufgebaut sein, die ein Metall, eine Mischung verschiedener Metalle, eine Metalllegierung, ein Metalloxid oder eine Mischung verschiedener Metalloxide enthält. Alternativ kann die Anode ein leitendes Polymer sein. Geeignete Metalle umfassen die Metalle der Gruppen Ib, IVa, Va und VIa des Periodensystems der Elemente sowie die Übergangsmetalle der Gruppe VIII. Wenn die Anode lichtdurchlässig sein soll, werden im Allgemeinen gemischte Metalloxide der Gruppen IIb, IIIb und IVb des Periodensystems der Elemente eingesetzt, zum Beispiel Indium-Zinn-Oxid (I-TO). Es ist ebenfalls möglich, dass die Anode (1) ein organisches Material, zum Beispiel Polyanilin enthält, wie beispielsweise in Nature, Vol. 357, Seiten 477 bis 479 (11. Juni 1992) beschrieben ist. Zumindest entweder die Anode oder die Kathode sollten mindestens teilweise transparent sein, um das gebildete Licht auskoppeln zu können.

Geeignete Lochtransportmaterialien für die Schicht (2) des erfindungsgemäßen OLEDs sind zum Beispiel in Kirk-Othmer Encyclopedia of Chemical Technologie, 4. Auflage, Vol. 18, Seiten 837 bis 860, 1996 offenbart. Sowohl Löcher transportierende Moleküle als auch Polymere können als Lochtransportmaterial eingesetzt werden. Üblicherweise eingesetzte Löcher transportierende Moleküle sind ausgewählt aus der Gruppe bestehend aus 4,4'-Bis[N-(1-naphthyl)-N-phenyl-amino]biphenyl (□-NPD), N,N'-Diphenyl-N,N'-bis(3-methylphenyl)-[1,1'-biphenyl]-4,4'-diamin (TPD), 1,1-Bis[(di-4-tolylamino)phenyl]cyclohexan (TAPC), N,N'-Bis(4-methylphenyl)-N,N'-Bis(4-ethylphenyl)-[1,1'-(3,3'-dimethyl)biphenyl]-4,4'-diamin (ETPD), Tetrakis-(3-methylphenyl)-N,N,N',N'-2,5-phenylendiamin (PDA), α-Phenyl-4-N,N-diphenyl-aminostyrol (TPS), p-(Diethylamino)-benzaldehyddiphenylhydrazon (DEH), Triphenylamin (TPA), Bis[4-(N,N-diethylamino)-2-methylphenyl)(4-methyl-phenyl)methan (MPMP), 1-Phenyl-3-[p-(diethylamino)styryl]-5-[p-(diethylamino)phenyl]pyrazolin (PPR oder DEASP), 1,2-trans-Bis(9H-carbazol-9-yl)cyclobutan (DCZB), N,N,N',N'-Tetrakis(4-methylphenyl)-(1,1'-biphenyl)-4,4'-diamin (TTB) und Porphyrinverbindungen wie Kupferphthalocyanine. Üblicherweise eingesetzte Löcher transportierende Polymere sind ausgewählt aus der Gruppe bestehend aus Polyvinylcarbazolen, (Phenylmethyl)polysilanen und Polyanilinen. Es ist ebenfalls möglich, Löcher transportierende Polymere durch Dotieren Löcher transportierender Moleküle in Polymere wie Polystyrol und Polycarbonat zu erhalten. Geeignete Löcher transportierende Moleküle sind die bereits vorstehend genannten Moleküle.

Geeignete Elektronen transportierende Materialien für die Schicht (4) der erfindungsgemäßen OLEDs umfassen mit oxinoiden Verbindungen chelatisierte Metalle wie Tris(8-chinolinolato)aluminium (Alq₃), Verbindungen auf Phenanthrolinbasis wie 2,9-Dimethyl,4,7-diphenyl-1,10-phenanthrolin (DDPA) oder 4,7-Diphenyl-1,10-phenanthrolin (DPA) und Azolverbindungen wie 2-(4-Biphenylyl)-5-(4-t-butylphenyl)-1,3,4-oxadiazol (PBD) und 3-(4-Biphenylyl)-4-phenyl-5-(4-t-butylphenyl)-1,2,4-triazol (TAZ). Dabei kann die Schicht (4) sowohl zur Erleichterung des Elektronentransports dienen als auch als Pufferschicht oder als Sperrschicht, um ein Quenchen des Excitons an den Grenzflächen der Schichten des OLEDs zu vermeiden. Vorzugsweise verbessert die Schicht (4) die Beweglichkeit der Elektronen und reduziert ein Quenchen des Excitons.

Die Kathode (5) ist eine Elektrode, die zur Einführung von Elektronen oder negativen Ladungsträgern dient. Die Kathode kann jedes Metall oder Nichtmetall sein, das eine geringere Arbeitsfunktion aufweist als die Anode. Geeignete Materialien für die Kathode sind ausgewählt aus der Gruppe bestehend aus Alkalimetallen der Gruppe Ia, zum Beispiel Li, Cs, Erdalkalimetallen der Gruppe IIa, Metallen der Gruppe IIb des Periodensystems der Elemente, umfassend die Seltenerdmetalle und die Lanthanide und Aktinide. Des Weiteren können Metalle wie Aluminium, Indium, Calcium, Barium, Samarium und Magnesium sowie Kombinationen davon eingesetzt werden. Weiterhin können Lithium enthaltende organometallische Verbindungen oder LiF zwischen der organischen Schicht und der Kathode aufgebracht werden, um die Betriebsspannung (Operating Voltage) zu vermindern.

Das OLED gemäß der vorliegenden Erfindung kann zusätzlich weitere Schichten enthalten, die dem Fachmann bekannt sind. Beispielsweise kann zwischen der Schicht (2) und der Licht emittierenden Schicht (3) eine Schicht aufgebracht sein, die den Transport der positiven Ladung erleichtert und/oder die Bänderlücke der Schichten aneinander anpasst. Alternativ kann diese weitere Schicht als Schutzschicht dienen. In analoger Weise können zusätzliche Schichten zwischen der Licht emittierenden Schicht (3) und der Schicht (4) vorhanden sein, um den Transport der negativen Ladung zu erleichtern und/oder die Bänderlücke zwischen den Schichten aneinander anzupassen. Alternativ kann diese Schicht als Schutzschicht dienen.

In einer bevorzugten Ausführungsform enthält das erfindungsgemäße OLED zusätzlich zu den Schichten (1) bis (5) mindestens eine der im Folgenden genannten weiteren Schichten:
- eine Loch-Injektionsschicht zwischen der Anode (1) und der Löcher-transportierenden Schicht (2);
- eine Blockschicht für Elektronen zwischen der Löcher-transportierenden Schicht (2) und der Licht-emittierenden Schicht (3);
- eine Blockschicht für Löcher zwischen der Licht-emittierenden Schicht (3) und der Elektronen-transportierenden Schicht (4);
- eine Elektronen-Injektionsschicht zwischen der Elektronen-transportierenden Schicht (4) und der Kathode (5).

Dem Fachmann ist bekannt, wie er (zum Beispiel auf Basis von elektrochemischen Untersuchungen) geeignete Materialien auswählen muss. Geeignete Materialien für die einzelnen Schichten sind dem Fachmann bekannt und z.B. in WO 00/70655 offenbart.

Des Weiteren kann jede der genannten Schichten des erfindungsgemäßen OLEDs aus zwei oder mehreren Schichten ausgebaut sein. Des Weiteren ist es möglich, dass einige oder alle der Schichten (1), (2), (3), (4) und (5) oberflächenbehandelt sind, um die Effizienz des Ladungsträgertransports zu erhöhen. Die Auswahl der Materialien für jede der genannten Schichten ist bevorzugt dadurch bestimmt, ein OLED mit einer hohen Effizienz zu erhalten.

Die Herstellung des erfindungsgemäßen OLEDs kann nach dem Fachmann bekannten Methoden erfolgen. Im Allgemeinen wird das OLED durch aufeinander folgende Dampfabscheidung (Vapor deposition) der einzelnen Schichten auf ein geeignetes Substrat hergestellt. Geeignete Substrate sind zum Beispiel Glas oder Polymerfilme. Zur Dampfabscheidung können übliche Techniken eingesetzt werden wie thermische Verdampfung, Chemical Vapor Deposition und andere. In einem alternativen Verfahren können die organischen Schichten aus Lösungen oder Dispersionen in geeigneten Lösungsmitteln beschichtet werden, wobei dem Fachmann bekannte Beschichtungstechniken angewendet werden.

Im Allgemeinen haben die verschiedenen Schichten folgende Dicken: Anode (2) 500 bis 5000 Å, bevorzugt 1000 bis 2000 Å; Löcher-transportierende Schicht (3) 50 bis 1000 Å, bevorzugt 200 bis 800 Å, Licht-emittierende Schicht (4) 10 bis 1000 Å, bevorzugt 100 bis 800 Å, Elektronen transportierende Schicht (5) 50 bis 1000 Å, bevorzugt 200 bis 800 Å, Kathode (6) 200 bis 10.000 Å, bevorzugt 300 bis 5000 Å. Die Lage der Rekombinationszone von Löchern und Elektronen in dem erfindungsgemäßen OLED und somit das Emissionsspektrum des OLED können durch die relative Dicke jeder Schicht beeinflusst werden. Das bedeutet, die Dicke der Elektronentransportschicht sollte bevorzugt so gewählt werden, dass die Elektronen/Löcher Rekombinationszone in der Licht-emittierenden Schicht liegt. Das Verhältnis der Schichtdicken der einzelnen Schichten in dem OLED ist von den eingesetzten Materialien abhängig. Die Schichtdicken von gegebenenfalls eingesetzten zusätzlichen Schichten sind dem Fachmann bekannt.

Durch Einsatz der erfindungsgemäß verwendeten Triazolderivate ausgewählt aus der Gruppe bestehend aus Triazolopyrimidinderivaten und Triazolouracilderivaten in der Licht-emittierenden Schicht der erfindungsgemäßen OLEDs können OLEDs mit hoher Effizienz erhalten werden. Die Effizienz der erfindungsgemäßen OLEDs kann des Weiteren durch Optimierung der anderen Schichten verbessert werden. Beispielsweise können hoch effiziente Kathoden wie Ca, Ba oder LiF eingesetzt werden. Geformte Substrate und neue Löcher-transportierende Materialien, die eine Reduktion der Operationsspannung oder eine Erhöhung der Quanteneffizienz bewirken, sind ebenfalls in den erfindungsgemäßen OLEDs einsetzbar. Des Weiteren können zusätzliche Schichten in den OLEDs vorhanden sein, um die Energielevel der verschiedenen Schichten einzustellen und um Elektrolumineszenz zu erleichtern.

Die erfindungsgemäßen OLEDs können in allen Vorrichtungen eingesetzt werden, worin Elektrolumineszenz nützlich ist. Geeignete Vorrichtungen sind bevorzugt ausgewählt aus stationären und mobilen Bildschirmen. Stationäre Bildschirme sind z.B. Bildschirme von Computern, Fernsehern, Bildschirme in Druckern, Küchengeräten sowie Reklametafeln, Beleuchtungen und Hinweistafeln. Mobile Bildschirme sind z.B. Bildschirme in Handys, Laptops, Fahrzeugen sowie Zielanzeigen an Bussen und Bahnen.

Weiterhin können die erfindungsgemäß eingesetzten Triazolderivate ausgewählt aus der Gruppe bestehend aus Triazolopyrimidinderivaten und Triazolouracilderivaten in OLEDs mit inverser Struktur eingesetzt werden. Bevorzugt werden die erfindungsgemäß eingesetzten Triazolderivate in diesen inversen OLEDs wiederum in der Licht-emittierenden Schicht, besonders bevorzugt als Licht-emittierende Schicht ohne weitere Zusätze, eingesetzt. Der Aufbau von inversen OLEDs und die üblicherweise darin eingesetzten Materialien sind dem Fachmann bekannt.

Einige der vorstehend genannten Triazolderivate der Formeln I, II, III und IV sind neue Verbindungen, die bisher nicht aus dem Stand der Technik bekannt sind. Ein weiterer Gegenstand der vorliegenden Erfindung sind daher Triazolderivate ausgewählt aus der Gruppe bestehend aus Triazolderivaten der allgemeinen Formeln I, II, III und IV 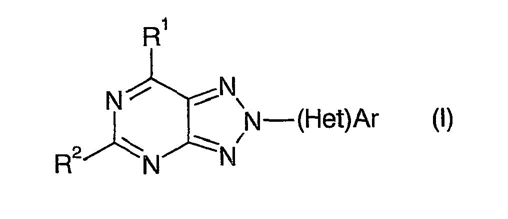 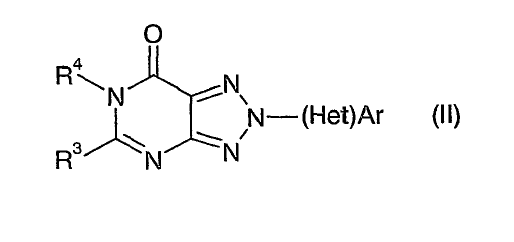 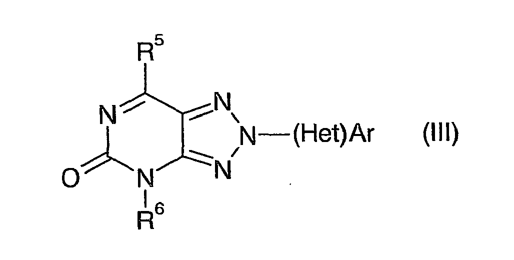 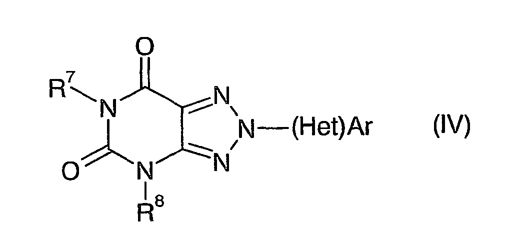 worin die Symbole die folgenden Bedeutungen aufweisen:
- R¹: Halogen, bevorzugt Cl, oder Amino, bevorzugt eine cyclische Aminogruppe, besonders bevorzugt Morpholino;
- R²: Amino, bevorzugt Dimethylamino;
- R³, R⁵: unabhängig voneinander Amino, bevorzugt Dimethylamino oder eine cyclische Aminogruppe, besonders bevorzugt Morpholino, Pyrrolidino oder Piperidino;
- R⁴, R⁶: unabhängig voneinander H, Alkyl, Aryl oder Heteroaryl, bevorzugt H;
- R⁷, R⁸: unabhängig voneinander H, Alkyl, Aryl, wobei R⁷ und R⁸ nicht gleichzeitig H bedeuten, bevorzugt Alkyl, besonders bevorzugt Methyl;
oder
- R³ und R⁴: bilden gemeinsam mit den Atomen, an die sie gebunden sind, einen 5- bis 7-gliedrigen Ring, der gegebenenfalls weitere Heteroatome, bevorzugt ausgewählt aus S, N und O, besonders bevorzugt ein weiteres N-Atom, enthält und gesättigt oder ungesättigt, bevorzugt gesättigt, ist, und unsubstituiert oder substituiert ist; besonders bevorzugt weist der Ring einen Substituenten ausgewählt aus Alkyl und Aryl an dem einen weiteren N-Atom auf, während die übrigen Atome des Rings unsubstituiert sind (d.h. Wasserstoffatome tragen);
- (Het)Ar: einen Rest der Formel 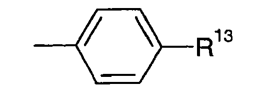
- R¹³: H, Alkyl, O-Alkyl, S-Alkyl, O-Aryl, S-Aryl oder Alkenylaryl, bevorzugt O-Alkyl, O-Phenyl, Phenyl, das substituiert oder unsubstituiert sein kann, oder Styryl, das unsubstituiert ist, besonders bevorzugt O-Alkyl oder Phenyl, das unsubstituiert ist, ganz besonders bevorzugt in den Verbindungen der Formeln I, II oder III O-CH₃ oder in den Verbindungen der Formel IV O-CH₃ oder unsubstituiertes Phenyl.

Die Bedeutungen der Begriffe Alkyl, Aryl, Heteroaryl, O-Alkyl, O-Aryl, S-Alkyl, S-Aryl, Halogen und Amino wurden bereits vorstehend erläutert.

Ganz besonders bevorzugt sind Triazolderivate der Formel I, worin (Het)Ar ein Rest der Formel 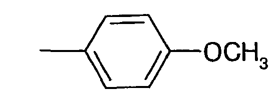 ist, und
R² Dimethylamino und R¹ Cl oder Morpholino bedeuten.

Des Weiteren sind Triazolderivate der Formel II ganz besonders bevorzugt, worin (Het)Ar ein Rest der Formel 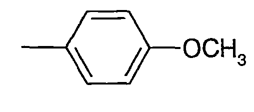 ist, und
R⁴ H und R³ ein Rest ausgewählt aus der Gruppe bestehend aus Dimethylamino, Morpholino, Pyrrolidino und Piperidino ist.

Des Weiteren sind Triazolderivate der Formel II ganz besonders bevorzugt, die die folgenden Formeln aufweisen: 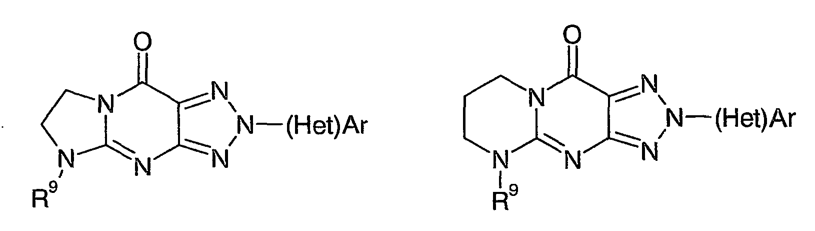 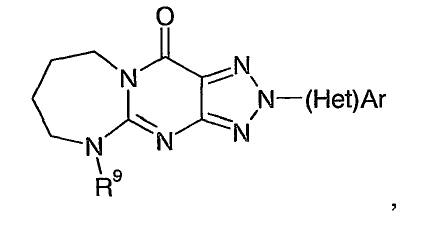 worin R⁹ Alkyl oder Aryl bedeutet und (Het)Ar ein Rest der Formel 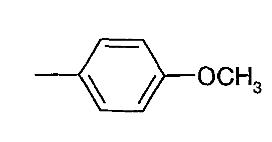 ist.

Weiterhin sind Triazolderivate der Formel IV ganz besonders bevorzugt, worin (Het)Ar ein Rest der Formel 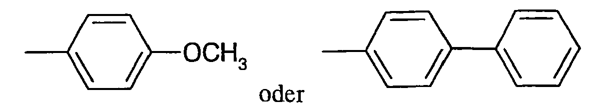 ist, und
R⁷ und R⁸ jeweils CH₃ bedeuten.

Die erfindungsgemäßen Triazolderivate der Formeln I, II, III und IV sind zum Einsatz in OLEDs, bevorzugt zum Einsatz in der Elektronen-leitenden Schicht und/oder in der Licht-emittierenden Schicht geeignet. Besonders bevorzugt werden die vorstehend genannten erfindungsgemäßen Triazolderivate als Emittersubstanzen in der Licht-emittierenden Schicht in OLEDs eingesetzt. Die Verwendung der erfindungsgemäßen Triazolderivate in OLEDs sowie OLEDs enthaltend die erfindungsgemäßen Triazolderivate und eine Licht-emittierende Schicht, enthaltend die erfindungsgemäßen Triazolderivate wurden, bereits vorstehend erläutert.

Die erfindungsgemäßen Triazolderivate können nach dem Fachmann bekannten Verfahren hergestellt werden. Geeignete Verfahren wurden ebenfalls bereits vorstehend erläutert.

Ein weiterer Gegenstand der vorliegenden Erfindung ist somit ein Verfahren zur Herstellung der erfindungsgemäßen Triazolderivate der allgemeinen Formeln I, II, III und IV wie vorstehend erwähnt, umfassend die Schritte:
a) Kupplung eines Amins der Formel V, VIII, X oder XII 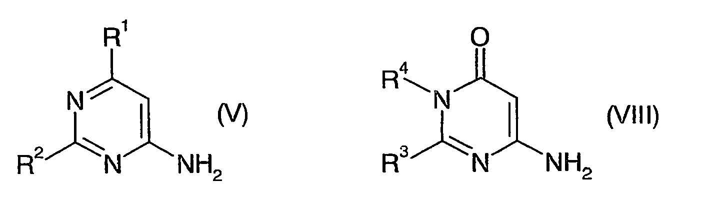 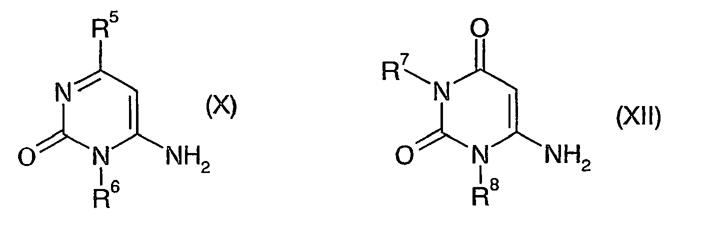
worin R¹, R², R³, R⁴, R⁵, R⁶, R⁷ und R⁸ die vorstehend bezüglich der erfindungsgemäßen Triazolderivate aufgeführten Bedeutungen aufweisen, mit einem Diazoniumsalz der Formel VI 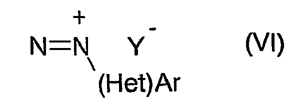 worin (Het)Ar die bezüglich der erfindungsgemäßen Triazolderivate angegebene Bedeutung aufweist und Y⁻ ein Anion bedeutet, wobei eine Azoverbindung der Formel VII, IX, XI oder XIII erhalten wird, 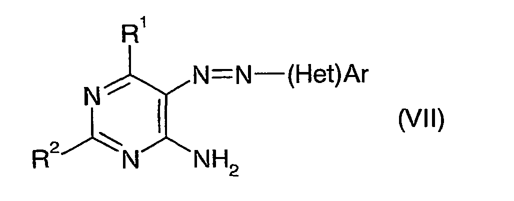 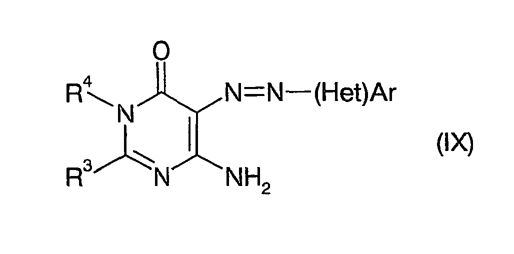 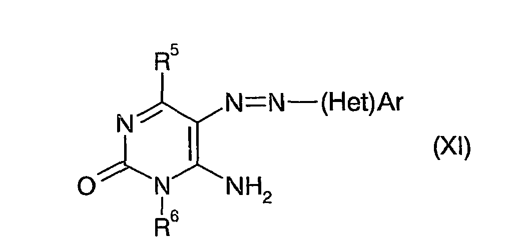 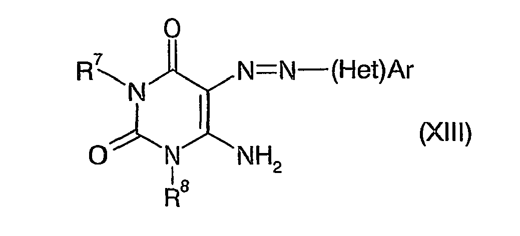 worin R¹, R², R³, R⁴, R⁵, R⁶, R⁷ und R⁸ sowie (Het)Ar die vorstehend bezüglich der erfindungsgemäßen Triazolderivate angegebenen Bedeutungen aufweisen;
b) oxidativer Ringschluss der Azoverbindungen der Formeln VII, IX, XI oder XIII, wobei die entsprechenden Triazolderivate der Formeln I, II, III oder IV gebildet werden.

In der Diazoniumverbindung der Formel VI ist das Anion Y⁻ bevorzugt ein Halogenid wie Chlorid oder Bromid, ein Sulfation oder ein Tetrafluoroboration.

Geeignete Reaktionsbedingungen der Verfahrensschritte a) und b) sind dieselben, die bereits vorstehend erläutert wurden. Die Herstellung der Ausgangsprodukte, die in den Schritten a) und b) eingesetzt werden, wurde ebenfalls vorstehend bereits erläutert und erfolgt im Allgemeinen in Analogie zu nach dem Fachmann bekannten Methoden.
Die nachfolgenden Beispiele erläutern die Erfindung zusätzlich.

### Beispiele

### Beispiel 1

### 5-Dimethylamino-2-(4-methoxyphenyl)-2H-[1,2,3]triazolo[4,5-d]pyrimidin-7-on

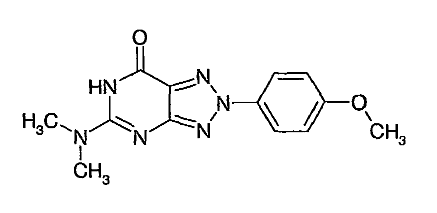

37.5 g (0.30 mol) 99 %iges p-Anisidin werden in 150 ml Wasser und 75 g konz. HCl suspendiert und auf 0-5°C gekühlt. Dazu wird eine Lösung von 20.7 g (0.30 mol) Natriumnitrit in 50 ml Wasser innerhalb von 40 min zugetropft. Die Reaktionslösung wird noch 30 min lang bei 0-5°C gerührt und anschließend zu einer Suspension von 46.3 g (0.30 mol) 6-Amino-5-dimethylamino-3H-pyrimidin-4-on und 24.6 g (0.30 mol ) Natriumacetat in 460 ml Wasser gegeben. Die Lösung wird 2 Stunden lang bei 0-5°C und über Nacht bei Raumtemperatur nachgerührt. Der Niederschlag wird abgesaugt, mit Wasser gewaschen und im Vakuum bei 75°C getrocknet. Es werden 74.4 g roter Azofarbstoff erhalten. Dieser wird in 904 ml Pyridin suspendiert. Die Suspension wird in eine 70°C heiße Lösung von 200 g (0.80 mol) Kupfer(II)-sulfat-pentahydrat in 904 ml Wasser innerhalb von 45 min getropft. Anschließend wird die Temperatur auf 90-100°C erhöht, wobei sich der Feststoff zunächst auflöst und sich dann ein gelbbrauner Niederschlag unter Aufschäumen der Suspension bildet. Die Suspension wird 15 Stunden lang bei 100°C gerührt und dann 6 Stunden lang mit Luft begast. Nach dem Abkühlen der Reaktionslösung wird der Niederschlag abgesaugt, mit Wasser gewaschen und bei 75°C im Vakuum getrocknet. Es werden 65.6 g Feststoff erhalten. Zur Reinigung wird eine Probe davon zweimal aus Dimethylformamid umkristallisiert und dann bei 250°C und 3x10⁻⁵ mbar sublimiert. Es wird ein analysenreiner fast farbloser Feststoff erhalten, der bei 311-312°C schmilzt.
- UV/Vis (DMSO):: □ₘₐₓ (lg □) = 348 nm (4.28)
□_{max,em} = 425 nm
IR (KBr): 1695, 1599, 1566, 1521, 1506, 1434, 1372, 1338,1279, 1250, 1222, 1169, 963 cm⁻¹

¹H-NMR (500 MHz; D₆-DMSO): 3.13 (s; 6H; N(CH₃)₂), 3.90 (s; 3H, OCH₃), 7.18 (d; 2H, Aromaten-H), 8.02 (d; 2H, Aromaten-H), 11.13 (s; 1H)
¹³C-NMR (500 MHz; D₆-DMSO): 37.96 (q; N-CH₃), 55.64 (q; OCH₃), 114.92 (d; Phenyl-C), 120.00 (d; Phenyl-C), 127.22 (s; quartäres C=N), 132.84 (s; N-Phenyl-C), 154.83 (s; quartäres C=N), 157.21 (s; O-Phenyl-C), 159.51 (s; C-N(CH₃)₂, C=O)

### Beispiel 2

### 5-Pyrrolidino-2-(4-methoxyphenyl)-2H-[1,2,3]triazolo[4,5-d]pyrimidin-7-on

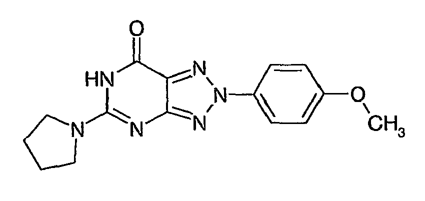

Die Herstellung erfolgt analog zu Beispiel 1 aus 5.41 g (30 mmol) 6-Amino-5-pyrrolidino-3H-pyrimidin-4-on. Es werden 6.45 g Azofarbstoff erhalten, der zu 4.13 g Triazolopyrimidinol (Rohprodukt) oxidiert wird. Zur Reinigung wird eine Probe davon in Dimethylformamid umkristallisiert.

Schmelzpunkt:. 329 - 332 °C (Zersetzung)
UV/Vis (DMSO): □ₘₐₓ (lg □) = 352 nm (4.27)
IR (KBr): 1705, 1691, 1612, 1592, 1565, 1521, 1507, 1458, 1438, 1403, 1248, 1014, 960, 835, 717 cm⁻¹

### Beispiel 3

### 5-Piperidino-2-(4-methoxyphenyl)-2H-[1,2,3]triazolo[4,5-d]pyrimidin-7-on

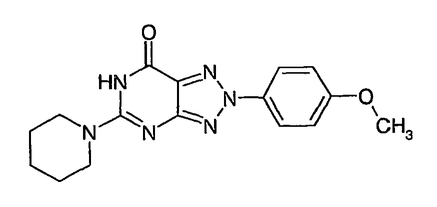

Die Herstellung erfolgt analog zu Beispiel 1 aus 2.94 g (15 mmol) 6-Amino-5-piperidino-3H-pyrimidin-4-on. Es werden 4.08 g Azofarbstoff erhalten, der zu 3.59 g Triazolopyrimidinol (Rohprodukt) oxidiert wird. Zur Reinigung wird eine Probe davon in Dimethylformamid umkristallisiert.

Schmelzpunkt 316 - 328 °C (Zersetzung)
UV/Vis (DMSO): □ₘₐₓ (lg □) = 348 nm (4.26)
IR (KBr): 1692, 1609, 1588, 1523, 1507, 1454, 1429, 1410, 1279, 1248, 1179, 1019, 963, 834 cm⁻¹

### Beispiel 4

### 5-Morpholino-2-(4-methoxyphenyl)-2H-[1,2,3]triazolo[4,5-d]pyrimidin-7-on

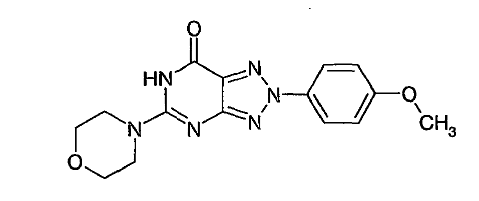

Die Herstellung erfolgt analog zu Beispiel 1 aus 4.79 g (30 mmol) 6-Amino-5-morpholino-3H-pyrimidin-4-on. Es werden 4.00 g Azofarbstoff erhalten, der zu 1.59 g Triazolopyrimidinol (Rohprodukt) oxidiert wird. Zur Reinigung wird eine Probe davon in Dimethylformamid umkristallisiert.

Schmelzpunkt 329 - 332°C (Zersetzung)
UV/Vis (DMSO): □ₘₐₓ (lg □) = 344 nm (4.29)
IR (KBr): 1691, 1609, 1587, 1524, 1507, 1469, 1447, 1369, 1281, 1250, 1116, 1017, 966, 833, 624 cm⁻¹

### Beispiel 5

### 7-Chlor-5-dimethylamino-2-(4-methoxyphenyl)-2H-[1,2,3]triazolo[4,5-d]pyrimidin

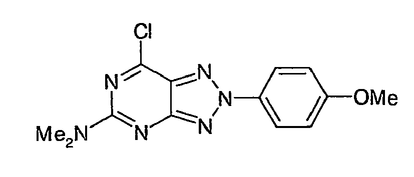

12.5 g (45 mmol) umkristallisiertes 5-Dimethylamino-2-(4-methoxyphenyl)-2H-[1,2,3]triazolo[4,5-d]pyrimidin-7-ol werden langsam in 56 ml Phosphoroxychlorid eingetragen. Die Reaktionsmischung wird 3 Stunden lang unter Rückfluss zum Sieden erhitzt. Anschließend wird Phosphoroxychlorid abdestilliert. Der Rückstand wird auf Eis gegeben. Die Suspension wird über Nacht gerührt, abgesaugt, mit Wasser gewaschen und im Vakuum bei 70°C getrocknet. Das Rohprodukt (12.39 g) wird in 150 ml Dioxan umkristallisiert. Es werden 10.16 g gelber Feststoff erhalten.

Schmelzpunkt 208-210°C

UV/Vis (Acetonitril): □ₘₐₓ (lg □) = 312 (3.99), 400 nm (4.20)
IR (KBr): 1613, 1595, 1566, 1548, 1537, 1507, 1413, 1366, 1258, 1214, 1181, 1042, 1027, 986, 829 cm⁻¹

¹H-NMR (500 MHz; CF₃CO₂D): 3.56 (s; 3H; NCH₃), 3.70 (s; 3H; NCH₃), 4.05 (s; 3H, OCH₃), 7.23 (d; 2H, Aromaten-H), 8.26 (d; 2H, Aromaten-H)
¹³C-NMR (500 MHz; CF₃CO₂D): 39.55 (q; N-CH₃), 41.50 (q; N-CH₃), 57.51 (q; OCH₃), 117.44 (d; Phenyl-C), 124.38 (d; Phenyl-C), 131.98 (s; quartäres C=N), 134.82 (s; N-Phenyl-C), 151.49 (s; C-Cl), 154.27 (s; O-Phenyl-C), 163.35 (s; quartäres C=N), 163.65 (s; C-N(CH₃)₂)

### Beispiel 6

### 5-Dimethylamino-7-morpholino-2-(4-methoxyphenyl)-2H-[1,2,3]triazolo[4,5-d]-pyrimidin

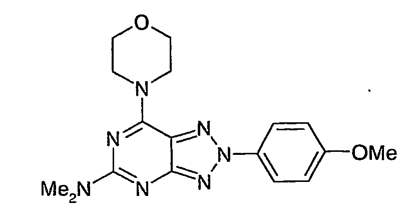

Zu einer Lösung von 1.50 g (4.92 mmol) 7-Chlor-5-dimethylamino-2-(4-methoxyphenyl)-2H-[1,2,3]triazolo[4,5-d]pyrimidin in 20 ml Ethanol werden 1.73 g (19.9 mmol) Morpholin bei Raumtemperatur unter Rühren zugetropft. Nach 45 min wird die Lösung mit 80 ml Wasser versetzt und noch 3 Stunden gerührt, wobei ein Niederschlag ausflockte. Dieser wird abgesaugt, mit Wasser gewaschen und im Vakuum bei 75°C getrocknet. Das Rohprodukt (1.71 g) wird zweimal in jeweils 20 ml Dioxan umkristallisiert. Es werden 0.65 g (37 %) hellgelbes Pulver isoliert.

Schmelzpunkt 220-227°C

UV/Vis (Methylenchlorid): □ₘₐₓ (lg □) = 258 (4.34), 290 (4.18), 374 nm (4.28)
(Chloroform): □_{max,em} = 449 nm
IR (KBr): 1583, 1574, 1532, 1506, 1446, 1400, 1388, 1359, 1247, 1230, 1160, 1111, 1021, 967, 835 cm⁻¹

¹H-NMR (500 MHz; CDCl₃): 3.25 (s; 6H; N(CH₃)₂), 3.88 (s; 3H, OCH₃), 3.89 (m; 4H, N-CH₂), 4.00 (breit; 2H, O-CH₂), 4.40 (breit; 2H, O-CH₂), 6.97 (d; 2H, Aromaten-H), 8.10 (d; 2H, Aromaten-H)
¹³C-NMR (500 MHz; CDCl₃): 37.48 (q; N-CH₃), 43.50 (t; N-CH₂), 47.50 (t; N-CH₂), 55.54 (q; OCH₃), 66.88 (t; O-CH₂), 114.27 (d; Phenyl-C), 120.84 (d; Phenyl-C), 123.60 (s; quartäres C=N), 133.61 (s; N-Phenyl-C), 153.80 (s; O-Phenyl-C), 159.51 (s; quartäres C=N), 160.95 (s; C-Morpholino), 162.23 (s; C-N(CH₃)₂)

### Beispiel 7

### 2-(4-Methoxyphenyl)-4,6-dimethyl-2H-[1,2,3]triazolo[4,5-d]uracil

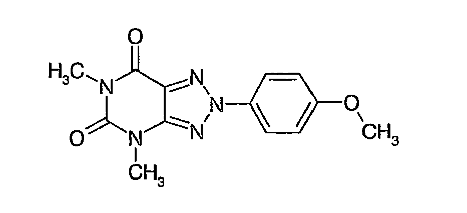

Zu einer Lösung von 4.34 g (15.0 mmol) 6-Amino-5-(4-methoxyphenylazo)-1,3-dimethyl-uracil in 52.5 ml Pyridin wird eine Lösung von 11.6 g (46.5 mmol) Kupfersulfat-pentahydrat in 52.5 ml Wasser bei 70°C innerhalb von 45 min unter Rühren zugetropft. Anschließend wird die Reaktionstemperatur auf 90-100°C erhöht. Die Reaktionslösung wird 15 Stunden lang bei dieser Temperatur gerührt und dann 6 Stunden lang mit Luft begast. Nach Abkühlen der Reaktionsmischung auf Raumtemperatur wird der entstandene Niederschlag abgesaugt, mit Wasser gewaschen und bei 75°C im Vakuum getrocknet. Das Rohprodukt (3.84 g) wird zweimal in jeweils 50 ml Dioxan umkristallisiert. 2.23 g (52 %) farbloser Feststoff werden isoliert.

Schmelzpunkt 221-223°C

UV/Vis (Methylenchlorid): □ₘₐₓ (lg □) = 326 nm (4.35)
(THF): □_{max,em} = 395 nm
IR (KBr): 1717, 1679, 1611, 1512, 1423, 1415, 1353, 1299, 1263, 1256, 1172, 1059, 1021, 838, 747 cm⁻¹

¹H-NMR (500 MHz; D₆-DMSO): 3.30 (s; 3H; NCH₃), 3.50 (s; 3H; NCH₃), 3.87 (s; 3H, OCH₃), 7.16 (d; 2H, Aromaten-H), 7.99 (d; 2H, Aromaten-H)
¹³C-NMR (500 MHz; D₆-DMSO): 27.93 (q; N-CH₃), 30.59 (q; N-CH₃), 55.47 (q; OCH₃), 114.79 (d; Phenyl-C), 120.49 (d; Phenyl-C), 125.89 (s; quartäres C=N), 132.03 (s; N-Phenyl-C), 149.71 (s; O-Phenyl-C), 150.61 (s; C=O), 155.43 (s; C=O), 159.58 (s; quartäres C=N)

### Beispiel 8

### 2-Biphenyl-4-yl-4,6-dimethyl-2H-[1,2,3]triazolo[4,5-d]uracil

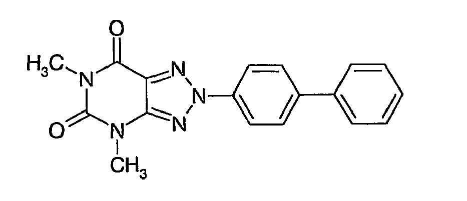

Zu einer Lösung von 20.1 g (60.0 mmol) 6-Amino-5-(biphenyl-4-ylazo)-1,3-dimethyl-uracil in 210 ml Pyridin wird eine Lösung von 47.2 g (189 mmol) Kupfersulfatpentahydrat in 210 ml Wasser bei 70°C innerhalb von 20 min unter Rühren zugetropft.

Anschließend wird die Reaktionstemperatur auf 90-100°C erhöht. Die Reaktionslösung wird 16.5 Stunden lang bei dieser Temperatur gerührt und dann 6 Stunden lang mit Luft begast. Nach Abkühlen der Reaktionsmischung auf Raumtemperatur wird der entstandene Niederschlag abgesaugt, mit Wasser gewaschen und bei 75°C im Vakuum getrocknet. Das Rohprodukt (14.36 g) wird mit Acetonitril extrahiert, wobei die Verunreingungen in Lösung gehen. Aus dem Rückstand werden nach dem Trocknen 8.18 g (41 %) gelblicher Feststoff erhalten.

Schmelzpunkt 247-250°C

UV/Vis (Methylenchlorid): □ₘₐₓ (lg □) = 330 nm (4.50)
(Chloroform): □_{max,em} = 402 nm
IR (KBr): 1723, 1685, 1610, 1603, 1556, 1548, 1522, 1490, 1423, 1414, 1353, 1299, 964, 766, 742 cm⁻¹

¹H-NMR (500 MHz; CDCl₃): 3.45 (s; 3H; NCH₃), 3.65 (s; 3H; NCH₃), 7.40 (m; 1H, Phenyl-H), 7.48 (m; 2H, Aromaten-H), 7.65 (m; 2H, Aromaten-H), 7.57 (m; 2H, Aromaten-H), 8.42 (m; 2H, Aromaten-H)
¹³C-NMR (500 MHz; CDCl₃/D₆-DMSO): 28.50 (q; N-CH₃), 31.03 (q; N-CH₃), 119.70 (d; Phenyl-C), 126.40 (s; quartäres C=N), 126.86 (d; Phenyl-C), 127.90 (d; Phenyl-C), 127.94 (d; Phenyl-C), 128.91 (d; Phenyl-C), 138.12 (s; N-Phenyl-C), 139.22 (s; Phenyl-C), 150.00 (s; C=O), 150.93 (s; C=O), 156.07 (s; quartäres C=N)

## Patentansprüche

1. Verwendung von Triazolderivaten ausgewählt aus der Gruppe bestehend aus Triazolopyrimidinderivaten und Triazolouracilderivaten in organischen Leuchtdioden.

2. Verwendung nach Anspruch 1, **dadurch gekennzeichnet, dass** die Triazolderivate als Emittermoleküle eingesetzt werden.

3. Verwendung nach Anspruch 1, **dadurch gekennzeichnet, dass** die Triazolderivate als Wirtsmoleküle in der Emitterschicht eingesetzt werden.

4. Verwendung nach einem der Ansprüche 1 bis 3, **dadurch gekennzeichnet, dass** die Triazolderivate ausgewählt sind aus Verbindungen der Strukturformeln I, II, III oder IV: 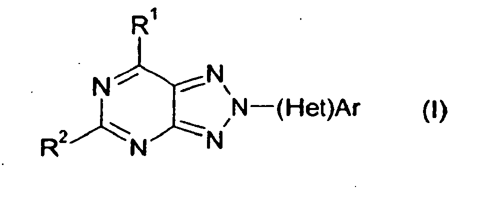 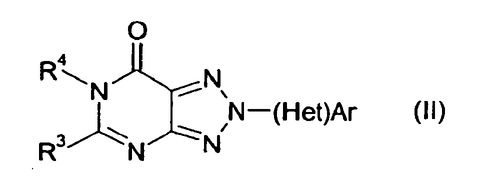 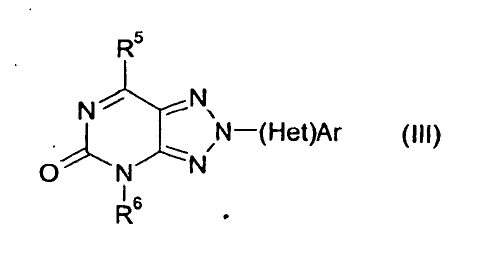 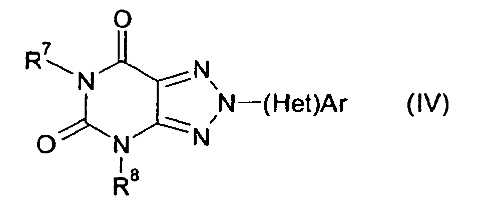 worin die Symbole die folgenden Bedeutungen aufweisen:
R¹, R², unabhängig voneinander H, Alkyl, Aryl, Heteroaryl, OH, O-Alkyl, O-Aryl, Halogen oder Amino, wobei mindestens einer der Substituenten R¹ oder R² OH, O-Alkyl, O-Aryl, Halogen oder Amino bedeutet;
R³, R⁵ unabhängig voneinander H, Alkyl, Aryl, Heteroaryl, OH, O-Alkyl, O-Aryl, Halogen oder Amino;
R⁴, R⁶, R⁷, R⁸ unabhängig voneinander H, Alkyl, Aryl oder Heteroaryl;
oder
R³ und R⁴ bilden gemeinsam mit den Atomen, an die sie gebunden sind, einen 4- bis 8-gliedrigen Ring, der gegebenenfalls weitere Heteroatome enthält und gesättigt oder ungesättigt und unsubstituiert oder substituiert ist;
(Het)Ar Aryl oder Heteroaryl.

5. Verwendung nach Anspruch 4, **dadurch gekennzeichnet, dass** der Rest (Het)Ar ein Rest der Formel 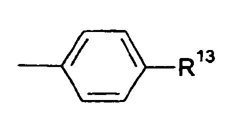 ist, worin
R¹³ H, Alkyl, O-Alkyl, S-Alkyl, Aryl, O-Aryl, S-Aryl oder Alkenylaryl bedeutet, bevorzugt H, OCH₃, Phenyl, das substituiert oder unsubstituiert sein kann, oder Styryl, das unsubstiuiert ist.

6. Verwendung nach Anspruch 4 oder 5, **dadurch gekennzeichnet, dass** in den Triazolderivaten der Formel 1
R¹ Cl, OH, OCH₃, OPh oder Morpholino bedeutet, bevorzugt OH, OCH₃, Cl oder Morpholino, besonders bevorzugt Cl oder Morpholino;
und/oder
R² OH, OCH₃, OPh, Piperidino, Pyrrolidino, Morpholino oder N(CH₃)₂ bedeutet, bevorzugt OH, N(CH₃)₂, Piperidino, Pyrrolidino oder Morpholino, besonders bevorzugt N(CH₃)₂.

7. Verwendung nach einem der Ansprüche 4 oder 5, **dadurch gekennzeichnet, dass** in den Triazolderivaten der Formeln II oder III
R³, R⁵ unabhängig voneinander OH, OCH₃, OPh, Piperidino, Pyrrolidino, Morpholino oder N(CH₃)₂ bedeuten, bevorzugt OH, N(CH₃)₂, Piperidino, Pyrrolidino oder Morpholino, besonders bevorzugt Piperidino, Pyrrolidino oder Morpholino;
und/oder
R⁴, R⁶ unabhängig voneinander H, CH₃ oder Phenyl bedeuten, bevorzugt H.

8. Verwendung nach einem der Ansprüche 4 oder 5, **dadurch gekennzeichnet, dass** in den Triazolderivaten der Formel II
R³ und R⁴ gemeinsam mit den Atomen, an die sie gebunden sind, einen 5- bis 7-gliedrigen Ring bilden, der gegebenenfalls weitere Heteroatome, bevorzugt ausgewählt aus S, N und O, besonders bevorzugt ein weiteres N-Atom, enthält und gesättigt oder ungesättigt, bevorzugt gesättigt, ist, und unsubstituiert oder substituiert ist; besonders bevorzugt weist der Ring einen Substituenten ausgewählt aus Alkyl und Aryl an dem einen weiteren N-Atom auf, während die übrigen Atome des Rings unsubstituiert sind.

9. Verwendung nach Anspruch 4 oder 5, **dadurch gekennzeichnet, dass** in den Triazolderivaten der Formel IV
R⁷und R⁸ unabhängig voneinander H, CH₃ oder Phenyl, bevorzugt CH₃ bedeuten.

10. Organische Leuchtdiode enthaltend mindestens ein Triazolderivat gemäß einem der Ansprüche 1 bis 9.

11. Licht-emittierende Schicht enthaltend mindestens ein Triazolderivat gemäß einem der Ansprüche 1 bis 9.

12. Organische Leuchtdiode enthaltend eine Licht-emittierende Schicht gemäß Anspruch 11.

13. Vorrichtung ausgewählt aus der Gruppe bestehend aus stationären Bildschirmen wie Bildschirmen von Computern, Fernsehern, Bildschirmen in Druckern, Küchengeräten sowie Reklametafeln, Beleuchtungen, Hinweistafeln und mobilen Bildschirmen wie Bildschirmen in Handys, Laptops, Fahrzeugen sowie Zielanzeigen an Bussen und Bahnen enthaltend ein OLED gemäß Anspruch 10 oder 12.

14. Triazolderivate der allgemeinen Formel I, II, III oder IV 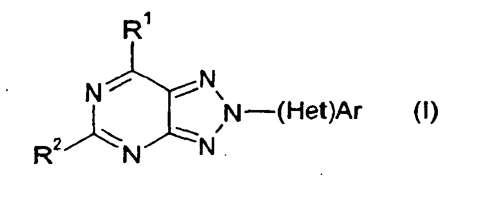 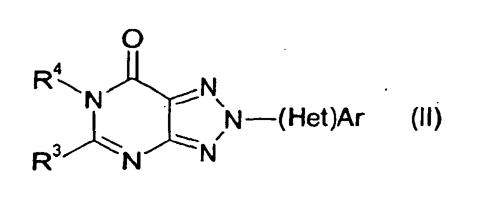 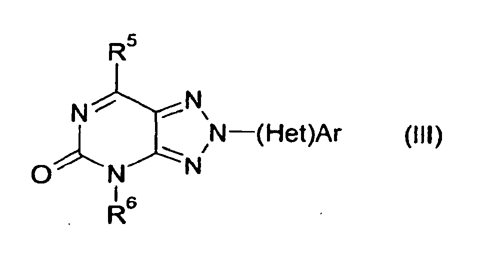 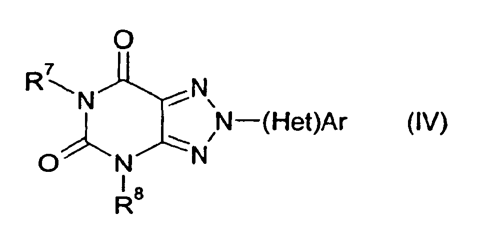 worin die Symbole die folgenden Bedeutungen aufweisen:
R¹ Halogen, bevorzugt Cl, oder eine cyclische Aminogruppe, besonders bevorzugt Morpholino;
R² Dimethylamino;
R³, R⁵ unabhängig voneinander Amino, bevorzugt Dimethylamino oder eine cyclische Aminogruppe, besonders bevorzugt Morpholino, Pyrrolidino oder Piperidino;
R⁴, R⁶ unabhängig voneinander H, Alkyl, Aryl oder Heteroaryl, bevorzugt H;
R⁷, R⁸ unabhängig voneinander H, Alkyl, Aryl, wobei R⁷ und R⁸ nicht gleichzeitig H bedeuten, bevorzugt Alkyl, besonders bevorzugt Methyl;
oder
R³ und R⁴ gemeinsam mit den Atomen, an die sie gebunden sind, einen 5- bis 7-gliedrigen Ring bilden, der gegebenenfalls weitere Heteroatome, bevorzugt ausgewählt aus S, N und O, besonders bevorzugt ein weiteres N-Atom, enthält und gesättigt oder ungesättigt, bevorzugt gesättigt, ist, und unsubstituiert oder substituiert ist; besonders bevorzugt weist der Ring einen Substituenten ausgewählt aus Alkyl und Aryl an dem einen weiteren N-Atom auf, während die übrigen Atome des Rings unsubstituiert sind;
(Het)Ar einen Rest der Formel 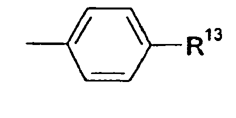
R¹³ H, Alkyl, O-Alkyl, S-Alkyl, Aryl, O-Aryl, S-Aryl oder Alkenylaryl, bevorzugt O-Alkyl, O-Phenyl, Phenyl, das substituiert oder unsubstituiert sein kann, oder Styryl, das unsubstituiert ist, besonders bevorzugt O-Alkyl oder Phenyl, das unsubstituiert ist, ganz besonders bevorzugt in den Verbindungen der Formeln I, II oder III O-CH₃ oder in den Verbindungen der Formel IV O-CH₃ oder unsubstituiertes Phenyl.

15. Verfahren zur Herstellung von Triazolderivaten der allgemeinen Formeln I, II, III und IV gemäß Anspruch 14, umfassend die Schritte:
a) Kupplung eines Amins der Formeln V, VIII, X oder XII 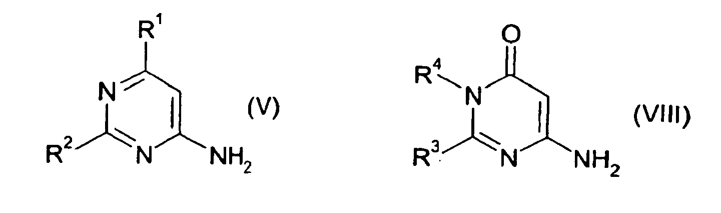 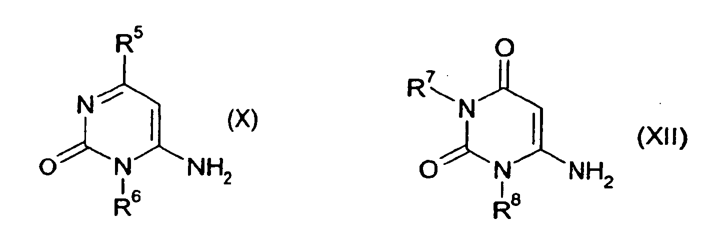 mit einem Diazoniumsalz der Formel VI 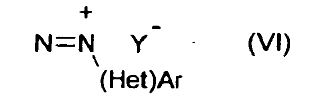 wobei eine Azoverbindung der Formel VII, IX, XI oder XIII erhalten wird; 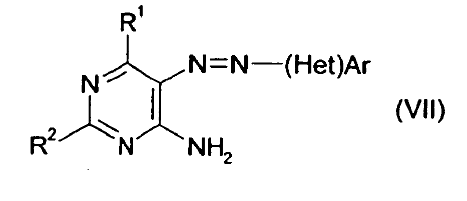 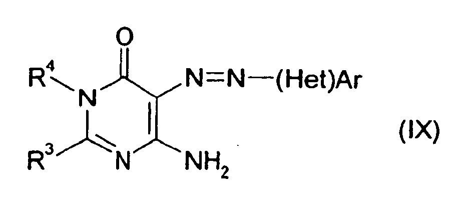 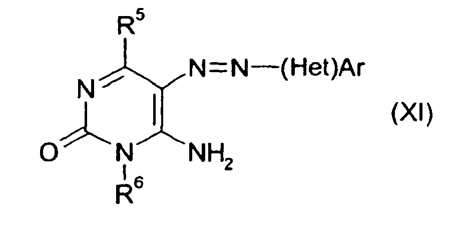 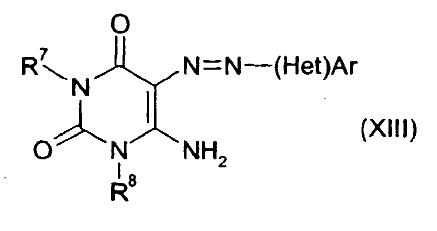 und
b) oxidativer Ringschluss der Azoverbindung der Formel VII, IX, XI oder XI-II, wobei das entsprechende Triazolderivat der Formel I, II, III oder IV gebildet wird;
worin die Symbole die folgenden Bedeutungen aufweisen:
R¹ Halogen, bevorzugt Cl, oder eine cyclische Aminogruppe, besonders bevorzugt Morpholino;
R² Dimethylamino;
R³, R⁵ unabhängig voneinander Amino, bevorzugt Dimethylamino oder eine cyclische Aminogruppe, besonders bevorzugt Morpholino, Pyrrolidino oder Piperidino;
R⁴, R⁶ unabhängig voneinander H, Alkyl, Aryl oder Heteroaryl, bevorzugt H;
R⁷, R⁸ unabhängig voneinander H, Alkyl, Aryl, wobei R⁷ und R⁸ nicht gleichzeitig H bedeuten, bevorzugt Alkyl, besonders bevorzugt Methyl;
oder
R³ und R⁴ gemeinsam mit den Atomen, an die sie gebunden sind, einen 5- bis 7-gliedrigen Ring bilden, der gegebenenfalls weitere Heteroatome, bevorzugt ausgewählt aus S, N und O, besonders bevorzugt ein weiteres N-Atom, enthält und gesättigt oder ungesättigt, bevorzugt gesättigt, ist, und unsubstituiert oder substituiert ist; besonders bevorzugt weist der Ring einen Substituenten ausgewählt aus Alkyl und Aryl an dem einen weiteren N-Atom auf, während die übrigen Atome des Rings unsubstituiert sind;
(Het)Ar einen Rest der Formel 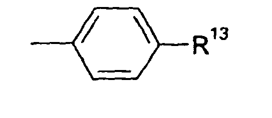
R¹³ H, Alkyl, O-Alkyl, S-Alkyl, Aryl, O-Aryl, S-Aryl oder Alkenylaryl, bevorzugt O-Alkyl, O-Phenyl, Phenyl, das substituiert oder unsubstituiert sein kann, oder Styryl, das unsubstituiert ist, besonders bevorzugt O-Alkyl oder Phenyl, das unsubstituiert ist, ganz besonders bevorzugt in den Verbindungen der Formeln I, II oder III O-CH₃ oder in den Verbindungen der Formel IV O-CH₃ oder unsubstituiertes Phenyl;
und
Y⁻ ein Anion, bevorzugt ein Halogenid wie Chlorid oder Bromid, ein Sulfation oder ein Tetrafluorboration.

## Claims

1. The use of triazole derivatives selected from the group consisting of triazolopyrimidine derivatives and triazolouracil derivatives in organic light-emitting diodes.

2. The use according to claim 1, wherein the triazole derivatives are used as emitter molecules.

3. The use according to claim 1, wherein the triazole derivatives are used as host molecules in the emitter layer.

4. The use according to any of claims 1 to 3, wherein the triazole derivatives are selected from among compounds of the structural formulae I, II, III or IV: 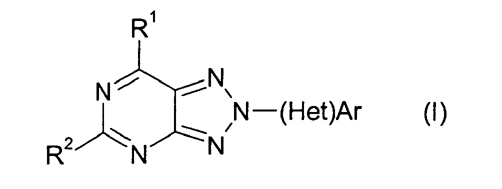 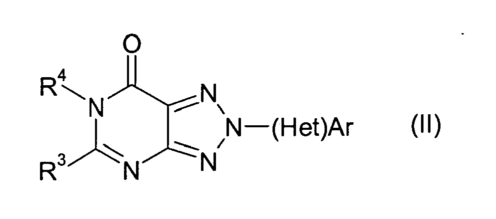 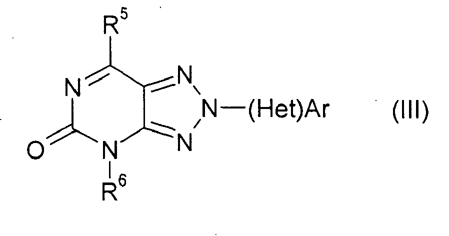 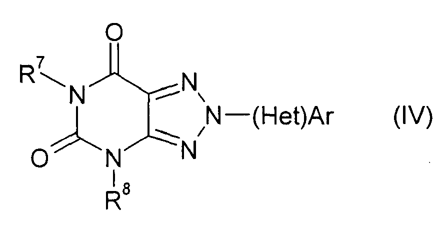 where the symbols have the following meanings:
R¹, R² are each, independently of one another, H, alkyl, aryl, heteroaryl, OH, O-alkyl, O-aryl, halogen or amino, with at least one of the substituents R¹ or R² being OH, O-alkyl, O-aryl, halogen or amino;
R³, R⁵ are each, independently of one another, H, alkyl, aryl, heteroaryl, OH, O-alkyl, O-aryl, halogen or amino;
R⁴, R⁶, R⁷, R⁸ are each, independently of one another, H, alkyl, aryl or heteroaryl;
or
R³ and R⁴ together with the atoms to which they are bound form a 4- to 8-membered ring which may comprise further heteroatoms and is saturated or unsaturated and unsubstituted or substituted;
(Het)Ar is aryl or heteroaryl.

5. The use according to claim 4, wherein the radical (Het)Ar is a radical of the formula 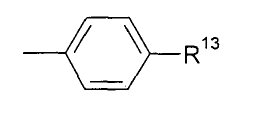 where
R¹³ is H, alkyl, O-alkyl, S-alkyl, aryl, O-aryl, S-aryl or alkenylaryl, preferably H, OCH₃, phenyl which may be substituted or unsubstituted or styryl which is unsubstituted.

6. The use according to claim 4 or 5, wherein, in the triazole derivatives of the formula I
R¹ is Cl, OH, OCH₃, OPh or morpholino, preferably OH, OCH₃, Cl or morpholino, particularly preferably Cl or morpholino;
and/or
R² is OH, OCH₃, OPh, piperidino, pyrrolidino, morpholino or N(CH₃)₂, preferably OH, N(CH₃)₂, piperidino, pyrrolidino or morpholino, particularly preferably N(CH₃)₂.

7. The use according to claim 4 or 5, wherein, in the triazole derivatives of the formula II or III,
R³, R⁵ are each, independently of one another, OH, OCH₃, OPh, piperidino, pyrrolidino, morpholino or N(CH₃)₂, preferably OH, N(CH₃)₂, piperidino, pyrrolidino or morpholino, particularly preferably piperidino, pyrrolidino or morpholino;
and/or
R⁴, R⁶ are each, independently of one another, H, CH₃ or phenyl, preferably H.

8. The use according to claim 4 or 5, wherein, in the triazole derivatives of the formula II,
R³ and R⁴ together with the atoms to which they are bound form a 5- to 7-membered ring which may comprise further heteroatoms, preferably selected from among S, N and O, particularly preferably a further N atom, and is saturated or unsaturated, preferably saturated, and is unsubstituted or substituted; the ring particularly preferably has a substituent selected from among alkyl and aryl on the one further N atom while the remaining atoms of the ring are unsubstituted.

9. The use according to claim 4 or 5, wherein, in the triazole derivatives of the formula IV
R⁷ and R⁸ are each, independently of one another, H, CH₃ or phenyl, preferably CH₃.

10. An organic, light-emitting diode comprising at least one triazole derivative according to any of claims 1 to 9.

11. A light-emitting layer comprising at least one triazole derivative according to any of claims 1 to 9.

12. An organic light-emitting diode comprising a light-emitting layer according to claim 11.

13. A device selected from the group consisting of stationary VDUs such as VDUs of computers, televisions, VDUs in printers, kitchen appliances and advertising signs, lighting, information signs and mobile VDUs such as VDUs in mobile telephones, laptops, vehicles and destination displays on buses and trains comprising an OLED according to claim 10 or 12.

14. A triazole derivative of the general formula I, II, III or IV 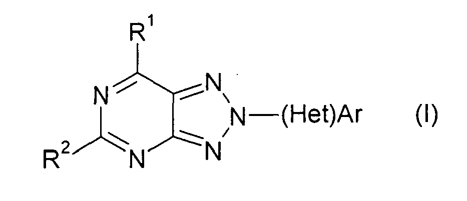 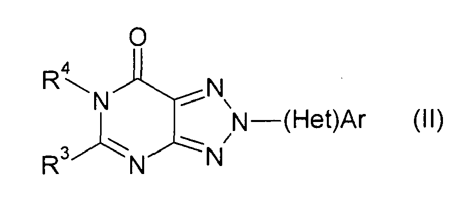 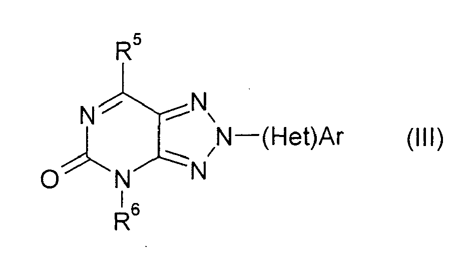 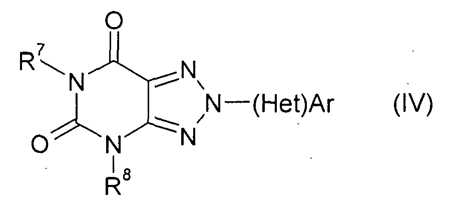 in which the symbols have the following meanings:
R¹ is halogen, preferably Cl, or a cyclic amino group, particularly preferably morpholino;
R² is dimethylamino;
R³, R⁵ are each, independently of one another, amino, preferably dimethylamino or a cyclic amino group, particularly preferably morpholino, pyrrolidino or piperidino;
R⁴, R⁶ are each, independently of one another, H, alkyl, aryl or heteroaryl, preferably H;
R⁷, R⁸ are each, independently of one another, H, alkyl, aryl, with R⁷ and R⁸ not both being H, preferably alkyl, particularly preferably methyl;
or
R³ and R⁴ together with the atoms to which they are bound form a 5- to 7-membered ring which may comprise further heteroatoms, preferably selected from among S, N and O, particularly preferably a further N atom, and is saturated or unsaturated, preferably saturated, and is unsubstituted or substituted; the ring particularly preferably has a substituent selected from among alkyl and aryl on the one further N atom while the remaining atoms of the ring are unsubstituted;
(Het)Ar is a radical of the formula 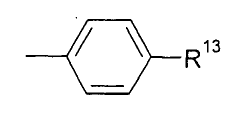
where
R¹³ is H, alkyl, O-alkyl, S-alkyl, aryl, O-aryl, S-aryl or alkenylaryl, preferably O-alkyl, O-phenyl, phenyl which may be substituted or unsubstituted or styryl which is unsubstituted, particularly preferably O-alkyl or phenyl which is unsubstituted, very particularly preferably in the compounds of the formulae I, II or III O-CH₃ or in the compounds of the formula IV O-CH₃ or unsubstituted phenyl.

15. A process for preparing triazole derivatives of the general formulae I, II, III and IV according to claim 14, which comprises the steps:
a) coupling of an amine of the formula V, VIII, X or XII 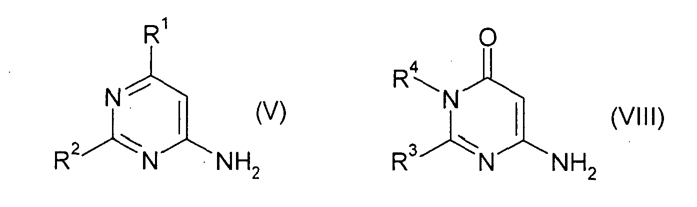 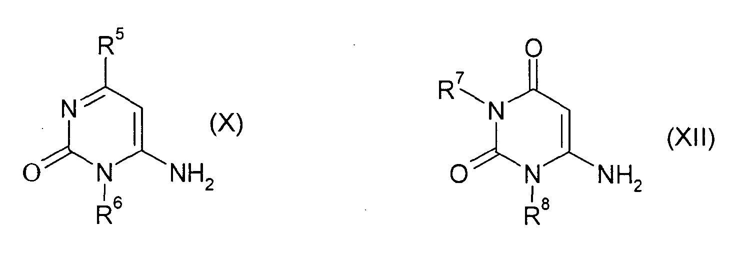 with a diazonium salt of the formula VI 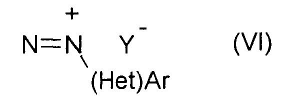 to give an azo compound of the formula VII, IX, XI or XIII; 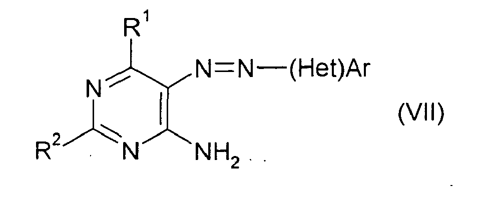 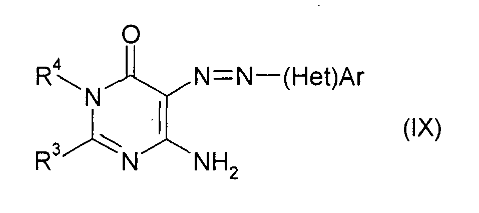 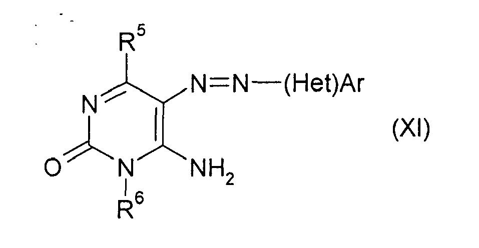 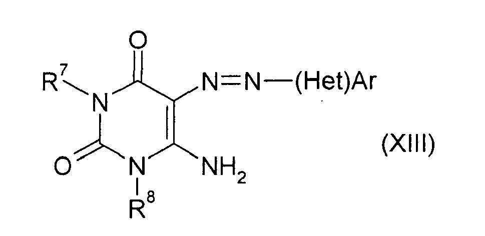 and
b) oxidative ring closure of the azo compound of the formula VII, IX, XI or XIII, to form the corresponding triazole derivative of the formula I, II, III or IV;
where the symbols have the following meanings:
R¹ is halogen, preferably Cl, or a cyclic amino group, particularly preferably morpholino;
R² is dimethylamino;
R³, R⁵ are each, independently of one another, amino, preferably dimethylamino or a cyclic amino group, particularly preferably morpholino, pyrrolidino or piperidino;
R⁴, R⁶ are each, independently of one another, H, alkyl, aryl or heteroaryl, preferably H;
R⁷, R⁸ are each, independently of one another, H, alkyl, aryl, with R⁷ and R⁸ not both being H, preferably alkyl, particularly preferably methyl;
or
R³ and R⁴ together with the atoms to which they are bound form a 5- to 7-membered ring which may comprise further heteroatoms, preferably selected from among S, N and O, particularly preferably a further N atom, and is saturated or unsaturated, preferably saturated, and is unsubstituted or substituted; the ring particularly preferably has a substituent selected from among alkyl and aryl on the one further N atom while the remaining atoms of the ring are unsubstituted;
(Het)Ar is a radical of the formula 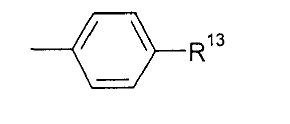
where
R¹³ is H, alkyl, O-alkyl, S-alkyl, aryl, O-aryl, S-aryl or alkenylaryl, preferably O-alkyl, O-phenyl, phenyl which may be substituted or unsubstituted or styryl which is unsubstituted, particularly preferably O-alkyl or phenyl which is unsubstituted, very particularly preferably in the compounds of the formulae I, II or III O-CH₃ or in the compounds of the formula IV O-CH₃ or unsubstituted phenyl;
and
Y⁻ is an anion, preferably a halide such as chloride or bromide, a sulfate ion or a tetrafluoroborate ion.

## Revendications

1. Utilisation de dérivés de triazol choisis dans le groupe constitué des dérivés de triazolopyrimidine et des dérivés de triazolouracil dans des diodes électroluminescentes organiques.

2. Utilisation selon la revendication 1, **caractérisée en ce que** les dérivés de triazol sont utilisés en tant que molécules émettrices.

3. Utilisation selon la revendication 1, **caractérisée en ce que** les dérivés de triazol sont utilisés en tant que molécules hôtes.

4. Utilisation selon l'une quelconque des revendications 1 à 3, **caractérisée en ce que** les dérivés de triazol sont choisis parmi les composés de formules structurales I, II, III ou IV : 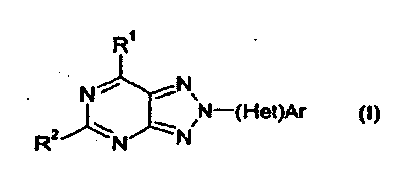 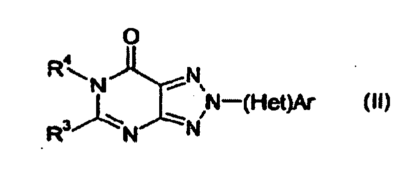 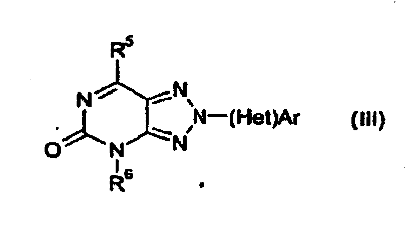 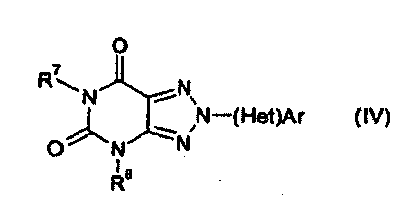 dans lesquelles les symboles ont les significations suivantes :
R¹, R² indépendamment l'un de l'autre, H, alkyle, aryle, hétéroaryle, OH, O-alkyle, O-aryle, halogène ou amino, au moins un des substituants R¹ ou R² signifiant OH, O-alkyle, O-aryle, halogène ou amino ;
R³, R⁵ indépendamment l'un de l'autre, H, alkyle, aryle, hétéroaryle, OH, O-alkyle, O-aryle, halogène ou amino ;
R⁴, R⁶, R⁷, R⁸ indépendamment les uns des autres, H, alkyle, aryle ou hétéroaryle ;
ou
R³ et R⁴ forment ensemble, avec les atomes auxquels ils sont reliés, un cycle à 4 à 8 éléments, qui contient éventuellement des hétéroatomes supplémentaires et qui est saturé ou insaturé et non substitué ou substitué ;
(Het)Ar aryle ou hétéroaryle.

5. Utilisation selon la revendication 4, **caractérisée en ce que** le radical (Het)Ar est un radical de formule 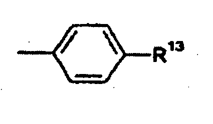 dans laquelle
R¹³ signifie H, alkyle, O-alkyle, S-alkyle, aryle, O-aryle, S-aryle ou alcénylaryle, de préférence H, OCH₃, phényle, qui peut être substitué ou non substitué, ou styryle, qui est non substitué.

6. Utilisation selon la revendication 4 ou 5, **caractérisée en ce que** dans les dérivés de triazol de formule I
R¹ signifie Cl, OH, OCH₃, OPh ou morpholino, de préférence OH, OCH₃, Cl ou morpholino, de manière particulièrement préférée Cl ou morpholino ;
et/ou
R² signifie OH, OCH₃, OPh, pipéridino, pyrrolidino, morpholino ou N(CH₃)₂, de préférence OH, N(CH₃)₂, pipéridino, pyrrolidino ou morpholino, de manière particulièrement préférée N(CH₃)₂.

7. Utilisation selon l'une quelconque des revendications 4 ou 5, **caractérisée en ce que** dans les dérivés de triazol de formules II ou III
R³, R⁵ signifient, indépendamment l'un de l'autre, OH, OCH₃, OPh, pipéridino, pyrrolidino, morpholino ou N(CH₃)₂, de préférence OH, N(CH₃)₂, pipéridino, pyrrolidino ou morpholino, de manière particulièrement préférée pipéridino, pyrrolidino ou morpholino ;
et/ou
R⁴, R⁶ signifient, indépendamment l'un de l'autre, H, CH₃ ou phényle, de préférence H.

8. Utilisation selon l'une quelconque des revendications 4 ou 5, **caractérisée en ce que** dans les dérivés de triazol de formules II
R³ et R⁴ forment ensemble, avec les atomes auxquels ils sont reliés, un cycle à 5 à 7 éléments, qui contient éventuellement des hétéroatomes supplémentaires, de préférence choisis parmi S, N et O, de manière particulièrement préférée un atome N supplémentaire, et qui est saturé ou insaturé, de préférence saturé, et qui est non substitué ou substitué ; de manière particulièrement préférée, le cycle comporte un substituant choisi parmi alkyle et aryle sur l'atome N supplémentaire, tandis que les autres atomes du cycle ne sont pas substitués.

9. Utilisation selon la revendication 4 ou 5, **caractérisée en ce que** dans les dérivés de triazol de formule IV
R⁷ et R⁸ signifient, indépendamment l'un de l'autre, H, CH₃ ou phényle, de préférence CH₃.

10. Diode électroluminescente organique contenant au moins un dérivé de triazol selon l'une quelconque des revendications 1 à 9.

11. Couche électroluminescente contenant au moins un dérivé de triazol selon l'une quelconque des revendications 1 à 9.

12. Diode électroluminescente organique contenant une couche électroluminescente selon la revendication 11.

13. Dispositif choisi dans le groupe constitué des écrans stationnaires tels que les écrans d'ordinateur, de télévision, les écrans d'imprimantes, les appareils de cuisine, ainsi que les panneaux publicitaires, les éclairages, les panneaux indicateurs, et les écrans mobiles tels que les écrans dans les téléphones portables, ordinateurs portables, véhicules, ainsi que les indicateurs de destination dans les bus et les trains, contenant une OLED selon la revendication 10 ou 12.

14. Dérivés de triazol de formule générale I, II, III ou IV 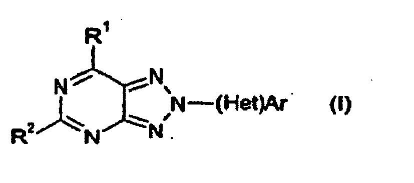 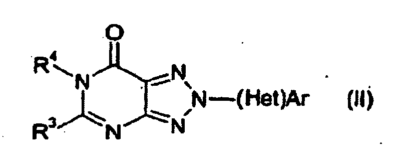 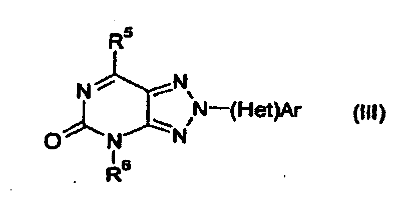 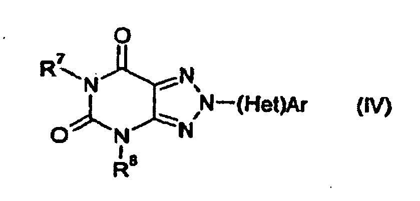 dans lesquelles les symboles ont les significations suivantes :
R¹ halogène, de préférence Cl, ou un groupe amino cyclique, de manière particulièrement préférée morpholino ;
R² diméthylamino ;
R³, R⁵ indépendamment l'un de l'autre, amino, de préférence diméthylamino ou un groupe amino cyclique, de manière particulièrement préférée morpholino, pyrrolidino ou pipéridino,
R⁴, R⁶ indépendamment l'un de l'autre, H, alkyle, aryle ou hétéroaryle, de préférence H ;
R⁷, R⁸ indépendamment l'un de l'autre, H, alkyle, aryle, R⁷ et R⁸ ne signifiant pas en même temps H, de préférence alkyle, de manière particulièrement préférée méthyle ;
ou
R³ et R⁴ forment ensemble, avec les atomes auxquels ils sont reliés, un cycle à 5 à 7 éléments, qui contient éventuellement des hétéroatomes supplémentaires, de préférence choisis parmi S, N et O, de manière particulièrement préférée un atome N supplémentaire, et qui est saturé ou insaturé, de préférence saturé, et qui est non substitué ou substitué ; de manière particulièrement préférée, le cycle comporte un substituant choisi parmi alkyle et aryle sur l'atome N supplémentaire, tandis que les autres atomes du cycle ne sont pas substitués ;
(Het)Ar un radical de formule 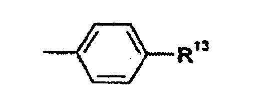
dans laquelle
R¹³ H, alkyle, O-alkyle, S-alkyle, aryle, O-aryle, S-aryle ou alcénylaryle, de préférence O-alkyle, O-phényle, phényle, qui peut être substitué ou non substitué, ou styryle, qui est non substitué, de manière particulièrement préférée O-alkyle ou phényle, qui n'est pas substitué, de manière tout particulièrement préférée dans les composés de formules I, II ou III O-CH₃ ou dans les composés de formule IV O-CH₃ ou phényle non substitué.

15. Procédé de fabrication de dérivés de triazol de formules générales I, II, III et IV selon la revendication 14, comprenant les étapes :
a) couplage d'une amine dé formule V, VIII, X ou XII 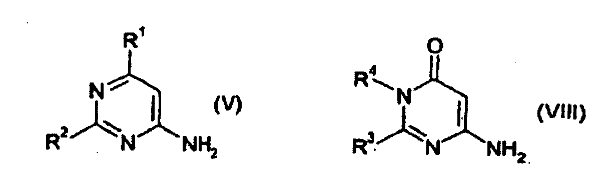 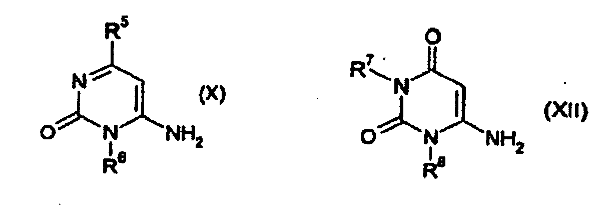 avec un sel de diazonium de formule VI 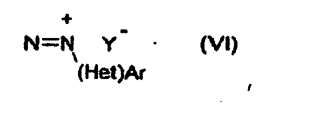 un composé azo de formule VII, IX, XI ou XIII étant obtenu ; 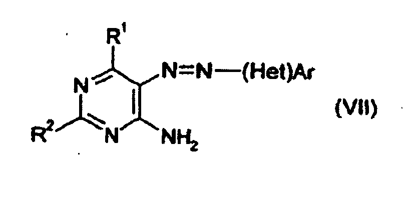 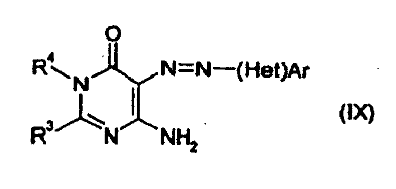 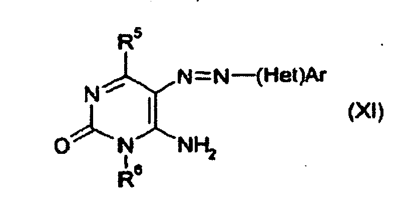 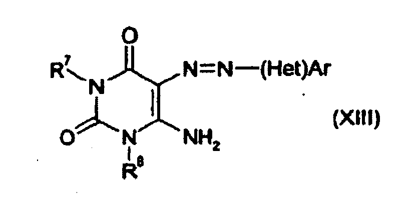 et
b) fermeture de cycle oxydative du composé azo de formule VII, IX, XI ou XIII, le dérivé de triazol correspondant de formule I, II, III ou IV se formant ;
les symboles ayant les significations suivantes :
R¹ halogène, de préférence Cl, ou un groupe amino cyclique, de manière particulièrement préférée morpholino ;
R² diméthylamino ;
R³, R⁵ indépendamment l'un de l'autre, amino, de préférence diméthylamino ou un groupe amino cyclique, de manière particulièrement préférée morpholino, pyrrolidino ou pipéridino,
R⁴, R⁶ indépendamment l'un de l'autre, H, alkyle, aryle ou hétéroaryle, de préférence H ;
R⁷, R⁸ indépendamment l'un de l'autre, H, alkyle, aryle, R⁷ et R⁸ ne signifiant pas en même temps H, de préférence alkyle, de manière particulièrement préférée méthyle ;
ou
R³ et R⁴ forment ensemble, avec les atomes auxquels ils sont reliés, un cycle à 5 à 7 éléments, qui contient éventuellement des hétéroatomes supplémentaires, de préférence choisis parmi S, N et O, de manière particulièrement préférée un atome N supplémentaire, et qui est saturé ou insaturé, de préférence saturé, et qui est non substitué ou substitué ; de manière particulièrement préférée, le cycle comporte un substituant choisi parmi alkyle et aryle sur l'atome N supplémentaire, tandis que les autres atomes du cycle ne sont pas substitués ;
(Het)Ar un radical de formule 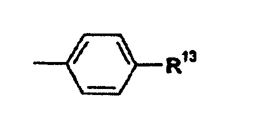
R¹³ H, alkyle, O-alkyle, S-alkyle, aryle, O-aryle, S-aryle ou alcénylaryle, de préférence O-alkyle, O-phényle, phényle, qui peut être substitué ou non substitué, ou styryle, qui est non substitué, de manière particulièrement préférée O-alkyle ou phényle, qui n'est pas substitué, de manière tout particulièrement préférée dans les composés de formules I, II ou III O-CH₃ ou dans les composés de formule IV O-CH₃ ou phényle non substitué ;
et
Y⁻ un anion, de préférence un halogénure tel que chlorure ou bromure, un ion sulfate ou un ion tétrafluoroborate.
